(19) Europäisches Patentamt / European Patent Office / Office européen des brevets

(11) **EP 3 727 337 B1**

(12) **EUROPEAN PATENT SPECIFICATION**

(45) Date of publication and mention
of the grant of the patent:
**16.07.2025  Bulletin 2025/29**

(21) Application number: **18839727.7**

(22) Date of filing: **21.12.2018**

(51) International Patent Classification (IPC):
**A61K 9/107** $^{(2006.01)}$   **A61K 9/127** $^{(2025.01)}$
**A61K 9/51** $^{(2006.01)}$   **A61K 31/573** $^{(2006.01)}$

(52) Cooperative Patent Classification (CPC):
**A61K 9/1075; A61K 9/127; A61K 9/51;
A61K 31/573**

(86) International application number:
**PCT/IB2018/060522**

(87) International publication number:
**WO 2019/123417 (27.06.2019 Gazette 2019/26)**

(54) **MICROEMULSION FOR OPTHALMIC DRUG DELIVERY**

MIKROEMULSION ZUR OPHTHALMISCHEN ARZNEIMITTELABGABE

MICRO-ÉMULSION POUR L'ADMINISTRATION OPHTALMIQUE DE MÉDICAMENT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.12.2017  GB 201721840**

(43) Date of publication of application:
**28.10.2020  Bulletin 2020/44**

(60) Divisional application:
**25181502.3**

(73) Proprietor: **Waterford Institute Of Technology
Waterford (IE)**

(72) Inventors:
• **BEHL, Gautam
Waterford (IE)**
• **KUMARI, Sangeeta
Waterford (IE)**

• **O'REILLY, Niall
Waterford (IE)**
• **O'DONOVAN, Orla
Waterford (IE)**
• **MCLOUGHLIN, Peter
Waterford (IE)**
• **KENT, David
Waterford (IE)**
• **FITZHENRY, Laurence
Waterford (IE)**

(74) Representative: **Barker Brettell LLP
100 Hagley Road
Edgbaston
Birmingham, West Midlands B16 8QQ (GB)**

(56) References cited:
**WO-A2-2012/103038    CN-A- 102 793 672
CN-A- 104 188 937    US-A1- 2017 296 496**

EP 3 727 337 B1

**Description**

The invention relates to a composition for ophthalmic delivery of a therapeutic agent, and the use of the composition in the treatment or prevention of ocular conditions.

Recent advances in the field of ophthalmic biotechnology resulted in a wide interest in a number of commercially available ophthalmic products. In recent years academic researchers have published a considerable body of work in the nanotherapeutics arena. Drug loaded nanoparticles have also been researched at preclinical and clinical stages. However, it has been quite challenging to develop an effective eye drop-based formulation that can overcome existing obstacles i.e. rapid and extensive precorneal loss caused by drainage and high tear fluid turnover, along with corneal and conjunctival epithelia acting as natural barriers further limiting the absorption of the drug. Generally frequent application of highly concentrated eye drop solution is required, due to the poor bioavailability (~2-7%) of topically applied ophthalmic drugs leading to absorption of drug systemically via the conjunctiva and nasolacrimal duct leading to unavoidable side effects.

Topical applications of corticosteroid are generally prescribed for conjunctivitis, anterior uveitis, dacryocystitis, keratitis and episcleritis and are also indicated for inflammation after cataract surgery and corneal operations. Among the corticosteroids, dexamethasone is reported to be the most potent and long acting glucocorticoid and has been one of the most widely used topically applied ocular therapeutic. Dexamethasone generally elicits its effect by binding to the corticosteroid receptors and inhibits the synthesis of prostaglandins and other agents primarily responsible for inflammation, thus decreasing the swelling and pain. However, the therapeutic effect of the drug gets compromised due to the stated lower bioavailability owing to the corneal and conjunctival epithelia barrier function. Dexamethasone is hydrophobic and faces extreme difficulty in penetration of the corneal epithelial barrier. The repeated application of dexamethasone eye drops to achieve therapeutic concentration further leads to high drug exposure causing serious side effects.

The majority of research addressing anterior segment ophthalmic disease has been focused on the development of eye drop formulations addressing dry eye disease/Keratoconjunctivitis Sicca. A decrease in aqueous tear secretion from the lacrimal gland is responsible for tear-deficient dry eye. The evaporative variant and most common form involving evaporation of the aqueous tear layer has been attributed to a deficient or unstable lipid layer outside protecting tear film. The lipid layer is responsible for controlling tear evaporation rate. The most common reason for this type is meibomian gland dysfunction (MGD), which may produce deficient lipids species in the tear lipid layer. Abnormal lipid composition affects the physicochemical properties of the tear film, and hence its stability. The primary and most recommended treatment is topical artificial tear replacement drops. However, most of these artificial tear drops (i.e. GenTeal®, Novartis Pharmaceuticals; Ultra Tears®, Alcon Laboratories, Inc.; Tearisol®, Novartis Pharmaceuticals; Lacril®, Allergan; Isopto®, Alcon Laboratories, Inc) do not provide lipid and only provide momentary relief to the patients, thus regular application is required causing further inconvenience and is cost ineffective. Most of these artificial eye drops are based on methylcellulose, polyvinyl alcohol, liquid polyol and their combinations.

Lipid-containing formulations have been provided to replace deficient lipid entities in the tear film induced by MGD and provide a medium to long acting tear replacement treatment. Refresh Endura (Allergan, Irvine, California) and Soothe (Bausch and Lomb, Rochester, New York) are examples of lipid-containing formulations in the market, which are described as having a longer-lasting lubricating effect while minimally interfering with the patient's vision [11]. Apart from these, lipid sprays containing liposomes i.e. Tears Again Liposome Lid Spray (OcuSoft, Richmond, Texas) and Liposic (Dr. Mann Pharma, Berlin, Germany) are available as an alternative to increase tear stability. Due to increased research and focus towards tear stabilisation newer lipid containing formulations i.e. Systane Balance (Alcon, Fort Worth, Texas) and Optive Advanced (Allergan, Irvine, California) have also emerged [13]. The lipid-containing Optrex range of eye products (Reckitt Benckiser, Inc.) are popular for use for dry eye disease. However all these lipid-containing formulations do not consider the role, and therapy, of the meibomian gland primarily responsible for lack of lipid layer protecting tear evaporation. Further these formulations do not address inflammation occurring due to dry eye syndrome. Therefore, repeated application is still required for continued relief.

Among medicated formulations for dry eye are Restasis™ (Allergan) which is an emulsion of cyclosporine [16]. Suspension based formulations are Maxidex (Alcon) and Lotemax (Bausch& Lomb) containing dexamethasone and loteprednol etabonate respectively [17-18]. Emulsion and suspension based formulations suffer from the disadvantage of toxicity of additives and blurred vision. Especially in the case of suspensions, the concentration of dissolved drug cannot be manipulated due to their relative insolubility in the vehicle [19].

Currently some of the approaches are being tried to prepare nanoparticulate formulations of corticosteroid: Lotepredenol etabonate, nanocrystals coated with a polymer as mucopenetrating formulation by (KP 121) Kala Pharmaceuticals [20-22] and dexamethasone-cylcodextrin nanoparticles (DexNP) by Oculis Pharma [23-24]. However, both of these formulations provide a nanoparticulate form of the drug without any matrix carrier, where their application can lead to direct exposure of ocular tissues to a high concertation of drug, leading to toxicity. Further, neither formulation addresses the problem associated with MGD.

Chinese Patent Application No: CN104188937A describes a montmorillonite-inlaid solid lipid nanoparticle preparation and a preparation method thereof. According to that invention, montmorillonite is selected as a drug carrier, and inlaid in a solid lipid nanoparticle, so that a drug sustained release preparation is prepared.

Chinese Patent Application No: CN102793672A describes a chitosan-modified methazolamide solid lipid nanoparticles and a preparation method thereof.

US Patent Application No: US2017296496A1 describes a water continuous dispersion of nanoparticles including Ibuprofen; one or more high HLB surfactants; one or more low HLB surfactants; and one or more oils. Methods of preparing various water continuous dispersion of nanoparticles are also provided therein.

An aim of the present invention is to provide an improved formulation for therapeutic relief or prevention of ocular conditions, such as dry eye.

According to a first aspect of the present invention, there is provided a composition for ophthalmic delivery of a therapeutic agent, the composition comprising an oil-in-water (o/w) microemulsion comprising a fatty acid, or fatty acid ester, as the oil phase; an aqueous phase; a surfactant; and a co-surfactant, and
wherein the composition further comprises a suspension of therapeutic agent-loaded nanoparticles in the aqueous phase of the oil-in-water emulsion.

The present invention advantageously provides a dual release platform comprising of microemulsion-nanoparticle blend containing fatty acid for meibomian gland regeneration and a therapeutic, such as an anti-inflammatory. The drug loading into the nanoparticles provides a means for its controlled release and maintaining an optimum level of drug at the target site, maintaining the desired therapeutic index. Liposome carrier systems based on membrane fusogenic lipid can be provided. The proposed technology not only shields the incorporated drug from physiological and chemical barriers within eye but also reduces the dosage frequency, increases the residence time and provides an enhanced therapeutic output. The advantages associated with the invention can include prolonged contact time with corneal tissue; mucofiltration technology to easily pass mucus barrier; simplicity of instillation for the patient; non-irritative and comfortable formulation; appropriate rheological properties; sterility; isotonicity; less drainage tendency; minimum protein binding; and capacity for use with a range of drug delivery systems, and a wide variety drugs used in various ophthalmic conditions.

In one embodiment, the fatty acid may comprise or consist of a saturated fatty acid, or an ester thereof. The saturated fatty acid may be selected from the group comprising lauric acid, myristic acid, capric acid, and tridecanoic acid and esters thereof; or combinations thereof. In one embodiment, the fatty acid may comprise or consist of an unsaturated fatty acid, or an ester thereof. The unsaturated fatty acid may be selected from the group comprising oleic acid, linoleic acid, linolenic acid, myristoleic acid, and palmitoleic acid, and esters thereof, or combinations thereof. The fatty acid ester may comprise of consist of an ethyl or methyl ester. E.g. an ethyl or methyl ester of any one or more of the saturated or unsaturated fatty acids herein described. A combination of saturated and unsaturated fatty acids, or esters thereof, may be provided. In one embodiment, the composition may comprise an oil-in-water (o/w) microemulsion comprising or consisting of an omega-3-fatty acid and/or omega-6-fatty acid as the oil phase. In one embodiment, the composition may comprise an oil-in-water (o/w) microemulsion comprising or consisting of an omega-3-fatty acid as the oil phase. In one embodiment, the omega-3-fatty acid and/or omega-6 fatty acid is derived from plant oils. In one embodiment, the omega-3-fatty acid comprises or consists of an omega-3-fatty acid selected from the group comprising α-linolenic acid, eicosapentaenoic acid (EPA), and docosahexaenoic acid (DHA); or combinations thereof. In one embodiment, the omega-3-fatty acid comprises or consists of α-linolenic acid. In one embodiment, the omega-6-fatty acid comprises or consists of linolenic acid. The skilled person will recognise that where an o/w emulsion is required, the selected fatty acid, or ester thereof, may be liquid in nature.

The oil phase, such as the omega-3-fatty acid and/or omega-6-fatty acid, may be provided in an amount of about 0.2% v/v of the composition. In another embodiment, the oil phase, such as the omega-3-fatty acid and/or omega-6-fatty acid, may be provided in an amount of between about 0.1% and about 0.5% v/v of the composition. In another embodiment, the oil phase, such as the omega-3-fatty acid and/or omega-6-fatty acid, may be provided in an amount of between about 0.15% and about 0.3% v/v of the composition. In another embodiment, the oil phase, such as the omega-3-fatty acid and/or omega-6-fatty acid, may be provided in an amount of between about 0.18% and about 0.22% v/v of the composition.

The aqueous phase of the composition may comprise water. In one embodiment, the water is deionised water. The composition may consist of at least about 95%, 96%, 97%, 98% or 99% water. In one embodiment the composition may consist of about 99% water. The skilled person will recognise the percentage of water will be determined by the total percentage of the other components of the composition, and may be adjusted up or down accordingly.

The surfactant may comprise any ophthalmically suitable molecule that can solubilise and reduce the interfacial tension between the oil and aqueous phases. The surfactant may be selected from the group comprising lecithin; lecithin derivatives; glycerol fatty acid esters; sorbitan fatty acid esters; polyoxyethylene sorbitan fatty acid esters; propylene glycol; and PEG 200; or combinations thereof.

A surfactant of lecithin or lecithin derivatives may comprise any of pure phospholipids, such as soya phosphatidyl choline; mixed phospholipids; sodium cholate and hydroxylated phospholipids/lecithin; or combinations thereof.

A surfactant of glycerol fatty acid ester may comprise any of polyglycerol fatty acid esters; polyglycerol polyricinoleate; propylene glycol fatty acid esters, such as polyoxyethylene glycerol triricinoleate; cremophor EL (macrogol-1500-glyceroltriricinoleate); monobutyl glycerol; or combinations thereof.

A surfactant of sorbitan fatty acid esters may comprise Span 20 (sorbitan monolaurate) and/or Span 80 (sorbitan monooleate).

A surfactant of polyoxyethylene sorbitan fatty acid esters may comprise Tween 20 (polyethylene glycol sorbitan monolaurate) and/or Tween 80 (polyethylene glycol sorbitan monooleate/polysorbate 80), and/or Labrasol (Polyethylene glycol-8-caprylic acid).

In one embodiment, the surfactant comprises or consists of polysorbate 80. Polysorbate 80 is advantageously a FDA approved non-ionic surfactant, which is used in a broad range of cosmetics and pharmaceutical formulations. It is biocompatible and an excellent stabiliser.

The surfactant, such as polysorbate 80, may be provided in an amount of between about 0.1 and about 2% v/v of the composition. In another embodiment, the surfactant, such as polysorbate 80, may be provided in an amount of between about 0.4 and about 1.5% v/v of the composition. In another embodiment, the surfactant, such as polysorbate 80, may be provided in an amount of between about 0.5 and about 1.2% v/v of the composition. In another embodiment, the surfactant, such as polysorbate 80, may be provided in an amount of between about 0.8 and about 1% v/v of the composition. In one embodiment, the surfactant, such as polysorbate 80, is provided in an amount of about 0.9% v/v of the composition.

The co-surfactant may comprise any ophthalmically suitable molecule that can promote the flexibility of the interface to promote the formation of the microemulsion. Preferably the co-surfactant is an ophthalmically suitable molecule that can penetrate into the interfacial film and produces a more fluid interface by allowing the hydrophobic tails of the surfactants to move freely at the interface. The co-surfactant may comprise or consist of an alkanol, such as ethanol, propanol, or 1-butanol; an alkane-diol, such as 1,2-Propane diol(propylene glycol) or 1,2-butane diol; an alkane-polyol, such as glycerol, glucitol, or polyethylene glycol, plurolisostearique (isosteric acid of polygelycerol), Plurololeique (Polyglyceryl-6-dioleate), Cremophor RH 40, or polyoxyethylene-10-oelyl ether (Brij 96V); PEG 200; and PEG 400; or combinations thereof. Co-surfactants may also comprise distearoylphosphatidyl ethanolamine-N-poly (ethyleneglycol) 2000 (DSPE-PEG), poloxamer and a combination where one component is block copolymer of a glycol monomer and other is polymer conjugated phospholipid. In one embodiment, the co-surfactant comprises or consists of PEG 400.

PEG 400 advantageously has good biocompatibility. It is non-irritating to ocular tissues and it is non-ionic and inert, such that it does not react with other formulation components. It also enhances the bioavailability for therapeutic agents having poor water solubility.

The co-surfactant, such as PEG 400, may be provided in an amount of between about 0.1 and about 2% v/v of the composition. In another embodiment, the co-surfactant, such as PEG 400, may be provided in an amount of between about 0.4 and about 1.5% v/v of the composition. In another embodiment, the co-surfactant, such as PEG 400, may be provided in an amount of between about 0.5 and about 1.2% v/v of the composition. In another embodiment, the co-surfactant, such as PEG 400, may be provided in an amount of between about 0.8 and about 1% v/v of the composition. In one embodiment, the co-surfactant, such as PEG 400, is provided in an amount of about 0.9% v/v of the composition.

The weight ratio of surfactant to co-surfactant may be from about 4:1 to about 1:2. The weight ratio of surfactant to co-surfactant may be any of about 1:1, about 2:1, about 3:1, about 4:1, or about 1:2; or any suitable ratio therebetween.

The weight ratio of oil phase (such as omega-3/-6 fatty acid) and surfactant/co-surfactant mixture may be between about 1.3 and about 1.9. The weight ratio of oil phase (such as omega-3/-6 fatty acid) and surfactant/co-surfactant mixture may be about 1:9, about 1:8, about 1:7, about 1:6, about 1:5, about 1:4, or about 1:3; or any suitable ratio therebetween.

In one embodiment, the nanoparticle may comprise a nanoparticle selected from the group comprising a liposome (i.e. an aqueous vesicle comprising at least one lipid bilayer); a nanoemulsion (i.e. a lipid monolayer with an oil core); a lipid nanocarrier (i.e. a solid lipid shell, and an oil core); a solid lipid nanoparticle (i.e. a solid lipid monolayer and a solid lipid core); a nanostructured lipid carrier (NLC) (i.e. lipid phase comprising a blend of solid and liquid lipids); a polymeric capsule (i.e. a solid polymer shell, and an oil or aqueous core); and a polymeric nanosphere (i.e. a solid polymer particle, such as a hydrogel); or combinations thereof. **In** one embodiment, the therapeutic agent-loaded nanoparticle is a liposome.

Advantageously, the use of a liposome provides a fusogenic delivery system that can efficiently deliver the therapeutic agent intracellularly. Other advantages of using liposome according to the present description are as follows:

1. Adsorption property of liposomes with corneal epithelium. In the presence of cell surface proteins, liposomes become leaky and release their contents in the vicinity of the cell membrane. This results in a higher concentration of drug in the vicinity of the cell membrane leading to cellular uptake of drug.

2. Endocytosis of the adsorbed liposomes by cell membrane thus finally releasing drug into the cytoplasm.

3. There is a unique similarity between liposomal membrane lipids and cell membrane, making liposomes to be recognized by lipid transfer protein present on cell membrane, consequently causing lipid exchange. This results in destabilisation of liposomal membrane and intracellular release of drug.

4. Fusion of liposomal membrane with cell membrane lipids by intermixing or lateral diffusion leading to direct delivery of drug in cytoplasm.

5. Liposomal lipids may also play a vital role in tear film stabilisation by improving the thickness of the lipid layer thus play an important role in therapy of dry eye syndrome.

6. All of the properties described above also serve as a means to prolong the retention of drug on the corneal surface.

7. Liposomal materials provide an intimate contact with corneal and conjunctival surface, thus enhancing corneal absorption of poorly water soluble drugs or drug with low partition coefficient.

8. Liposomal vesicular system also protects the drug at the corneal surface from degradation by enzymes present in tears or on the corneal epithelial surface.

9. Liposomal materials can improve pharmacokinetic profile, enhance therapeutic efficacy and reduce toxicity.

The above advantages are particularly effective for conditions such as dry eye syndrome, with the ability of liposomes to stabilise tear film, enhance drug permeation and reduce side effects. The use of liposomes loaded with drug specifically in combination with a fatty acid or fatty acid ester, such as $\alpha$-linolenic acid, provides the advantage of liposomes described above along with meibomian gland regeneration effect, and a therapeutic effect from the agent, such as the anti-inflammatory effect of dexamethasone. Other nanoparticle types can also be used suitably with the microemulsion depending on the type of therapeutic agent and stability of the final formulation

In an embodiment comprising the use of polymeric nanoparticles, the polymeric particles may comprise or consist of polymers selected from the group comprising sodium alginate, chitosan, poly (ethylene glycol), polylactide, polyacrylamide, gelatine, cellulose and its derivatives, polyacrylates, poloxamers, polycaprolactone, poly(N-vinyl caprolactam), polyethylenimine and their block copolymers or graft polymers; or combinations thereof.

In an embodiment comprising the use of lipid based nanoparticles such as SLN (solid lipid nanoparticles) and NLC's (nanostructured lipid carrier), polymeric capsules can be provided based on solid lipids, for example, selected from cetylpalmitate, glyceryl dibehenate (Compitrol 888 ATO) , glycerol monostearate, glycerol palmitostearate, palmitic acid, glyceryl palmitostearate (Precirol ATO 5), stearic acid, tripalmitin, tristearin, and cholesterol; or combinations thereof. Liquid lipids may also be provided, such as oleic acid, glyceryl tricaprylate/caprate (Miglyol 812) and castor oil, paraffin oil, 2-octyl dodecanol, propylene glycol dicaprylocaprate (Labrafac®), isopropyl myristate and squalene; or combinations thereof. Surfactant can be provided, such as Poly(ethylene glycol)-block-poly(propylene glycol)-block-poly(ethylene glycol) (Poloxamer 188), Polysorbate 20, Polysorbate 80, polyvinyl alcohol, sodium cholate, sodium glycocholate, sodium taurocholate, sodium trioleate, soyabean lecithin and soyabean phosphatidylcholine; or combinations thereof.

In one embodiment, the nanoparticles, such as liposomes, comprise vesicles prepared from phospholipid and another lipid, such as cholesterol, and it is loaded with the therapeutic agent. In one embodiment, the phospholipid comprises or consists of lecithin. The phospholipid may comprise soya lecithin, such as Phospholipon G®.

The phospholipid, such as lecithin or Phospholipon G®, may be provided in an amount of about 2.4% w/v of the composition. In another embodiment, the phospholipid, such as lecithin or Phospholipon G®, may be provided in an amount of between about 1% and about 5% v/v of the composition. In another embodiment, the phospholipid, such as lecithin or Phospholipon G®, may be provided in an amount of between about 2% and about 3% v/v of the composition.

In one embodiment, the nanoparticles, such as the liposomes, comprise another lipid, such as cholesterol. The liposome bilayer may comprise cholesterol. In another embodiment, the liposomes comprise a lipid selected from the group comprising tristearin, stearic acid, cetyl palmitate, cholesterol, glyceryl distearate NF / glyceryl palmitostearate (e.g. Precirol® ATO 5), esters of behenic acid with glycerol(e.g. Compritol® 888 ATO), tripalmitin (e.g. Dynasan®116), tristearin (e.g. Dynasan® 118), hydrogenated palm oil (e.g. Softisan® 154), cetylpalmitate (e.g. Cutina® CP), glyceryl stearate (e.g. Imwitor® 900 P), glycerol monostearate (e.g. Geleol®), glycerol monostearate and PEG-75 stearate (e.g. Gelot® 64), cetyl alcohol and ceteth-20 / steareth-20 (e.g. Emulcire® 61) and cholesterol; or combinations thereof. Reference to a branded molecule/composition may be used interchangeably with the generic form thereof.

The cell membrane comprises a lipid bilayer composed of phospholipids and cholesterol. The use of phospholipid and cholesterol in a liposome according to the description can provide better fusion with cell membrane via lipid exchange, endocytosis and adsorption.

The other lipid, such as cholesterol, may be provided in an amount of about 0.29% w/v of the composition. In another embodiment, the other lipid, such as cholesterol, may be provided in an amount of about 0.294% w/v of the composition. In another embodiment, the other lipid, such as cholesterol, may be provided in an amount of between about 0.1% and about 0.5% w/v of the composition. In another embodiment, the other lipid, such as cholesterol, may be provided in an amount of between about 0.2% and about 0.4% w/v of the composition. In another embodiment, the other lipid, such as cholesterol, may be provided in an amount of between about 0.25% and about 0.35% w/v of the composition.

The weight ratio of the phospholipid, such as lecithin, to the other lipid, such as cholesterol, may be between about 1:1 and about 5:1. The weight ratio of the phospholipid, such as lecithin, to the other lipid, such as cholesterol, may be about 1:1, about 2:1, about 3:1, about 4:1, or about 5:1; or any suitable ratio therebetween. In one embodiment, the weight ratio of the phospholipid, such as lecithin, to the other lipid, such as cholesterol, may be about 4:1.

The nanoparticles, such as liposomes, may be further coated with a polymer or copolymer. In one embodiment, the nanoparticles, such as liposomes comprise an amphiphilic block copolymer, such as a poloxamer. The poloxamer may comprise Pluronic F-127® (Also known as Poloxamer 407). Pluronic F-127® is a triblock copolymer consisting of a central hydrophobic block of polypropylene glycol flanked by two hydrophilic blocks of polyethylene glycol (PEG). The polymer coating, such as the poloxamer, may be inlaid in the surface and/or adsorbed on the surface of the nanoparticles, such as liposomes.

The provision of a polymer coating, such as a poloxamer, advantageously enhances stability, masking the charge, thus making the nanoparticles, such as liposomes, nearly neutral. It also helps the nanoparticles, such as liposomes, to permeate through mucin.

In one embodiment, the polymer coated nanoparticles, such as liposomes, are prepared with about a 1:1:5 molar ratio of phospholipid (such as lecithin or Phospholipon G): other lipid such as cholesterol: polymer (such as PF 127). In another embodiment, the ratio may be selected from about 4:1:1; 4:1:2.5; 4:1:4; 1:1:4; 1:1:1; and 1:1:2.5, or any ratios therebetween.

The liposomes may be of nanoparticle size. In one embodiment, the liposomes are less than about 1000nm at their average largest diameter. In another embodiment, the liposomes are less than about 800nm at their average largest diameter. In another embodiment, the liposomes are less than about 600nm at their average largest diameter. In another embodiment, the liposomes are less than about 500nm at their average largest diameter. In another embodiment, the liposomes are less than about 400nm at their average largest diameter. In another embodiment, the liposomes are less than about 300nm at their average largest diameter. In another embodiment, the liposomes are less than about 200nm at their average largest diameter. In another embodiment, the liposomes are less than about 100nm at their average largest diameter. In another embodiment, the liposomes are less than about 80nm at their average largest diameter. In another embodiment, the liposomes are less than about 50nm at their average largest diameter. In another embodiment, the liposomes are less than about 40nm at their average largest diameter. In another embodiment, the liposomes are about 30nm at their average largest diameter. In another embodiment, the liposomes are between about 15nm and about 1000nm at their average largest diameter. In another embodiment, the liposomes are between about 15nm and about 500nm at their average largest diameter. In another embodiment, the liposomes are between about 15nm and about 100nm at their average largest diameter. In another embodiment, the liposomes are between about 15nm and about 50nm at their average largest diameter. In another embodiment, the liposomes are between about 20nm and about 80nm at their average largest diameter. In another embodiment, the liposomes are between about 20nm and about 50nm at their average largest diameter. In another embodiment, the liposomes are between about 20nm and about 40nm at their average largest diameter. In another embodiment, the liposomes are between about 25nm and about 35nm at their average largest diameter.

The therapeutic agent may be hydrophobic. In one embodiment, the therapeutic agent is an anti-inflammatory agent. In one embodiment, the anti-inflammatory agent is dexamethasone. The anti-inflammatory agent may comprise or consist of a corticosteroid, such as dexamethasone. In another embodiment, the corticosteroid may be selected from the group comprising fluocinolone, difluprednate, loteprednol, fluorometholone, medrysone, dexamethasone, prednisolone, triamcinolone, and rimexolone, or combinations thereof. The therapeutic agent may comprise an antihistamine and/or decongestant.

The skilled person will recognise that hydrophobic therapeutic agents, such as dexamethasone, would be provided in the lipid bilayer of the liposomes.

The therapeutic agent may be provided in the amount of about 0.1 % w/v of the composition. In another embodiment, the therapeutic agent may be provided in the amount of between about 0.01 % and about 0.5 % w/v of the composition. In another embodiment, the therapeutic agent may be provided in the amount of between about 0.05 % and about 0.2 % w/v of the composition. In another embodiment, the therapeutic agent may be provided in the amount of less than about 0.5 % w/v of the composition. In another embodiment, the therapeutic agent may be provided in the amount of between about 0.1 % and about 0.2 % w/v of the composition.

In another embodiment, the therapeutic agent may be provided at a concentration of between about 0.01 mg/ml and about 2 mg/ml. In another embodiment, the therapeutic agent may be provided at a concentration of between about 0.01 mg/ml and about 1.5 mg/ml. In another embodiment, the therapeutic agent may be provided at a concentration of between about 0.01 mg/ml and about 1 mg/ml. In another embodiment, the therapeutic agent may be provided at a concentration of between about 0.05 mg/ml and about 1 mg/ml. In another embodiment, the therapeutic agent may be provided at a concentration of between about 0.1 mg/ml and about 1 mg/ml. In another embodiment, the therapeutic agent may be provided at a concentration of between about 0.2 mg/ml and about 1 mg/ml. In another embodiment, the therapeutic agent may be provided at a concentration of between about 0.5 mg/ml and about 1 mg/ml. In another embodiment, the therapeutic agent may be provided at a concentration of between about 0.01 mg/ml and about 0.5 mg/ml. In another embodiment, the therapeutic agent may be provided at a concentration of between about 0.01 mg/ml and about 0.2 mg/ml. In another embodiment, the therapeutic agent may be provided at a concentration of between about 0.5 mg/ml and about 1.5 mg/ml. In another embodiment, the therapeutic agent may be provided at a concentration of less than about 2 mg/ml.

The therapeutic agent may be provided in combination with one or more other therapeutically active agents. For example, a second, third or more therapeutic agent may be provided in the nanoparticles, oil phase or aqueous phase, or a combination thereof. The second, third or more therapeutic agent may comprise an antibiotic.

The composition may be a pharmaceutically acceptable composition. The composition may be an ophthalmically acceptable composition. The composition may be suitable for topical administration to the eye. **In** one embodiment, the composition is an ophthalmic composition. An ophthalmic composition is understood to be a sterile, liquid, semisolid, or solid preparation that may contain one or more active pharmaceutical ingredient(s) (i.e. the anti-inflammatory agent described herein) intended for application to the eye or eyelid.

The composition may comprise one or more ophthalmically acceptable ingredients selected from the group consisting of: water; saline; salt; buffer; demulcent; humectant; viscosity increasing agent; tonicity adjusting agent; cellulose derivatives e.g. carboxymethylcellulose sodium, hydroxyethyl cellulose, hydroxypropyl methylcellulose, or methylcellulose; dextran 70; gelatin; polyols; glycerine; polyethylene glycol e.g. PEG300 or PEG400; polysorbate 80; propylene glycol; polyvinyl alcohol; and povidone (polyvinyl pyrrolidone); and combinations thereof.

Demulcents may comprise or consist of cellulose derivatives, glycerine, polyvinyl alcohol, polyvinyl pyrrolidone, cellulose derivatives, polyethylene glycol, or combinations thereof.

**In** one embodiment, the therapeutic agent is suitable for treatment or prevention of an eye disorder. The eye disorder may comprise any one of the disorders selected from dry eye syndrome (keratoconjunctivitis sicca); conjunctivitis; keratitis; uveitis; scelritis; episcleritis; blepharitis; acanthamoeba keratitis; and iritis; or combinations thereof. In one embodiment, the therapeutic agent is suitable for treatment or prevention of dry eye.

Also described is a composition according to the description herein for use in the treatment or prevention of an eye disorder in a subject.

Also described is the use of a composition according to the description herein in the manufacture of a medicament for treatment or prevention of an eye disorder in a subject.

Also described is a method of treatment or prevention of an eye disorder in a subject comprising the administration of the composition according to the description to an eye of the subject.

The administration may be topical to the surface of the eye or to the eyelid.

The subject may be mammalian. **In** one embodiment, the subject is a human subject. The subject may be in need of treatment for the eye disorder or may be at risk of developing the eye disorder. **In** one embodiment, the subject is a non-human animal, such as a domestic or livestock animal. For example, the use may be veterinary.

The administration of the composition may be a pharmaceutically effective amount of the composition. The treatment or prevention may comprise a single administration or repeated administrations. The administration may be once every 1 to 18 days. **In** another embodiment, the administration may be once every 1 to 14 days. The administration may be once every 5 to 18 days. The administration may be once every 7 to 18 days. The administration may be once every 10 to 18 days. The administration may be once every 15 to 18 days. The administration may be about once every 7 days. The administration may be no more than once per day.

The eye disorder may be selected from dry eye syndrome (keratoconjunctivitis sicca), conjunctivitis, keratitis, uveitis, sceleritis, episcleritis, blepharitis, acanthamoeba keratitis, dacryostenosis, dacryocystitis, and iritis; or combinations thereof. The disorder may be acute or chronic.

The use or administration may be following eye surgery, for example after a cataract operation.

Also described is an eye drop dispenser or eye wash device comprising the composition according to the description herein.

An eye drop dispenser may otherwise be known as an eye drop applicator. Typical eye drop dispensers comprise a reservoir for the composition and an outlet for the composition. The outlet may be tapered towards a distal end, with the outlet orifice at the tip/distal end. The dispenser may be arranged to be sealed, for example with a cap. An eye drop dispenser may alternatively comprise a syringe device.

The term "nanoparticle" is intended to refer herein to a microscopic particle of matter that acts as a single unit, and that is measured on the nanoscale. Nanoparticles typically measure less than 100nm, for example 10-100nm, at their largest dimension. However, the present description may also encompass nanoparticles of up to 500nm or 1000nm. The skilled person will recognise that at scales of 500nm to 1000nm the nanoparticles may alternatively be termed "ultrafine particles" where appropriate.

A "microemulsion" is understood to be a thermodynamically stable mixture of immiscible fluids, such as oil and water, achieved by dividing one phase into very small droplets, which are typically 20-200nm in size. The description herein is directed a microemulsion of oil suspended in water or aqueous phase (o/w).

A liposome is understood to be an aqueous vesicle comprising at least one lipid bilayer. Liposomes are typically used as vehicles for administration of nutrients and pharmaceutical drugs.

The term "ophthalmic delivery of an agent" is understood to mean the provision of an agent, such as a drug, to tissues of the eye. Ophthalmic delivery of an agent may comprise external topical or droplet delivery to the eye, and it is not intended to mean systemic delivery through the blood stream.

The term "prevention" means avoidance of a disorder or a protective treatment for a disorder. The prevention may include a reduced risk of the disorder, reduced risk of infection, transmission and/or progression, or reduced severity of the disorder.

The term "treatment" means a cure of a condition or disease, an alleviation of symptoms, or a reduction in severity of a disorder or symptoms of the disorder.

The skilled person will understand that optional features of one embodiment or aspect of the invention may be applicable, where appropriate, to other embodiments or aspects of the invention.

Embodiments of the invention will now be described in more detail, by way of example only, with reference to the accompanying figures.

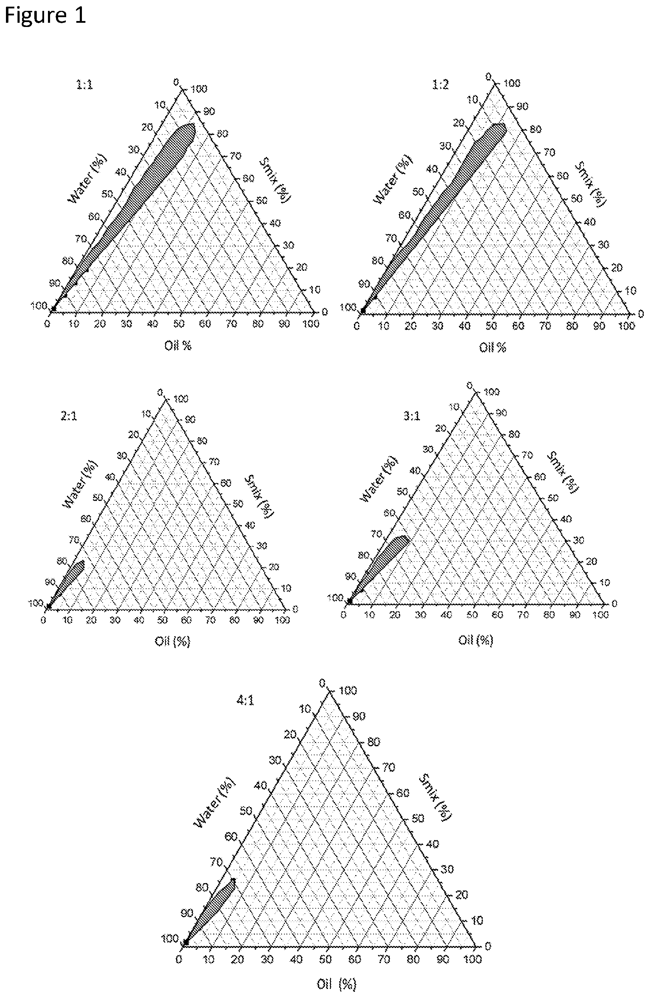

**Figure 1.** Pseudo ternary phase diagrams with the Smix ratios (Suf:Co-surf).

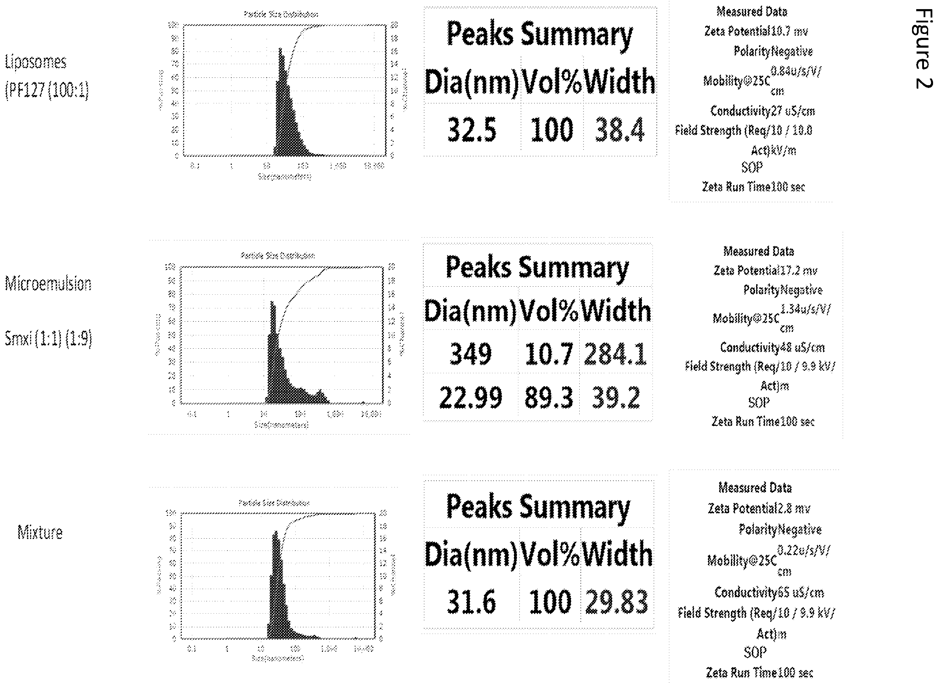

**Figure 2.** Microemulsion (Smix 1:1; oil:Smix 1:9) and PF 127 coated liposome mixing study (Lipid: PF127; 100:1).

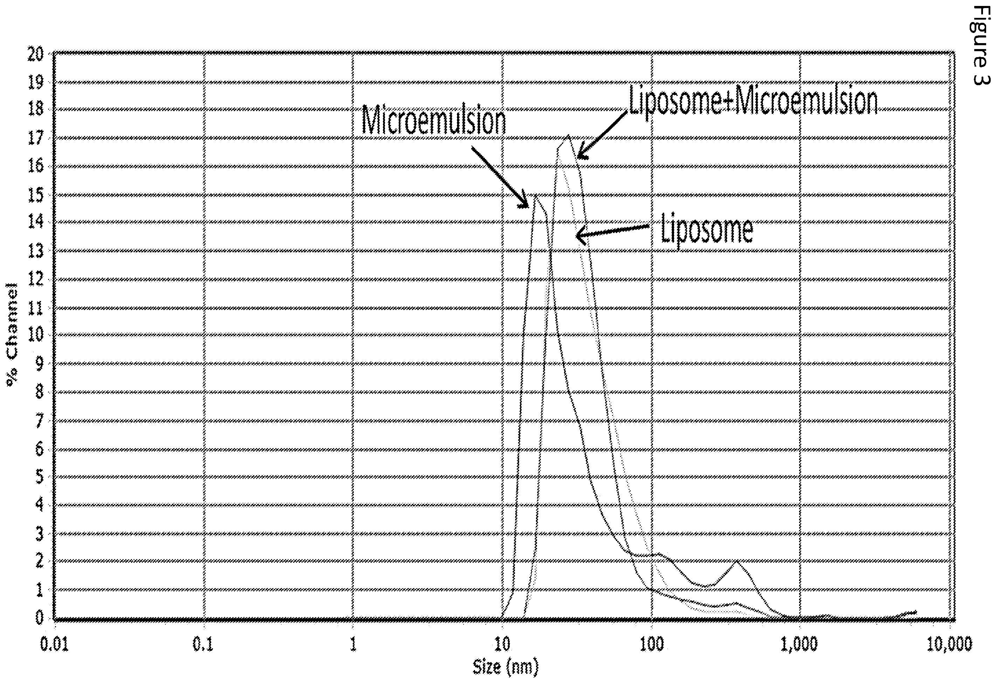

**Figure 3.** Comparative DLS study of microemulsion (Smix 1:1; oil: Smix 1:9)-liposome mixture (Lipid: PF127; 100:1).

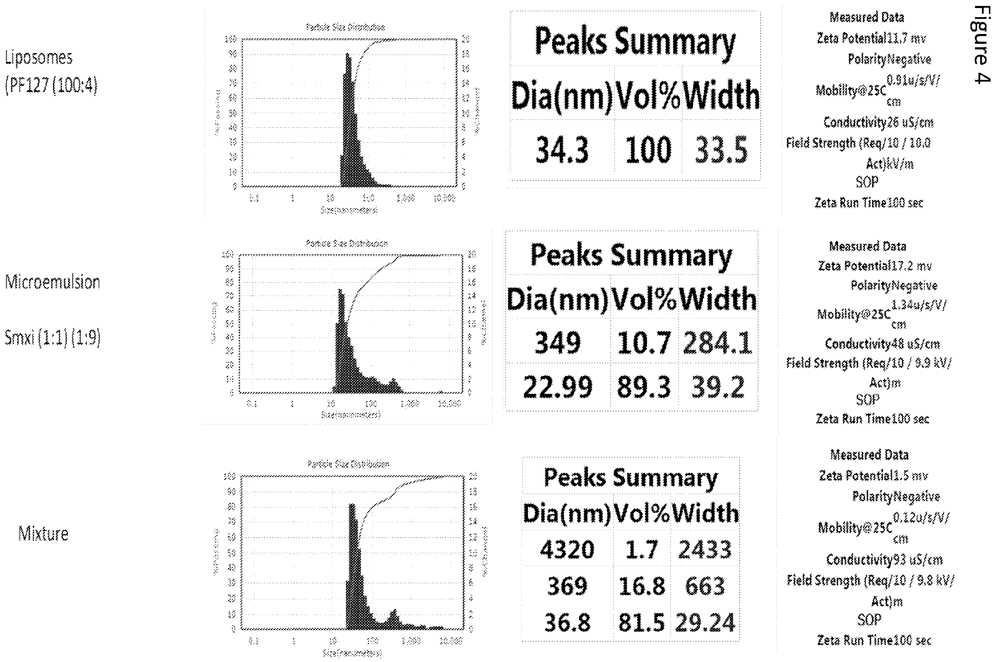

**Figure 4.** Microemulsion (Smix 1:1; oil:Smix 1:9) and PF 127 coated liposome mixing study (Lipid: PF127; 100:4).

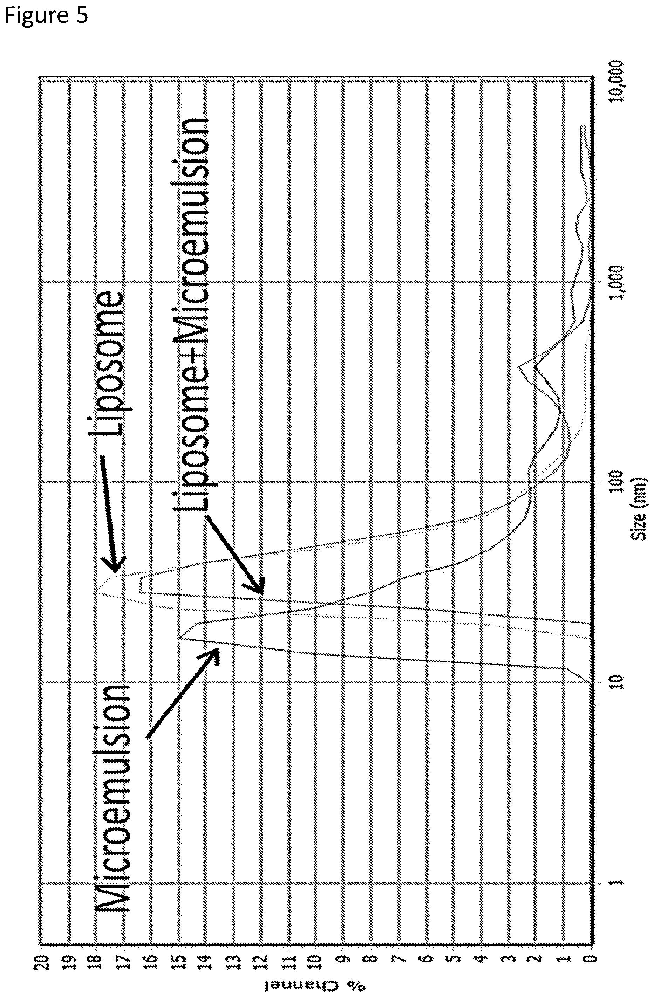

**Figure 5.** Comparative DLS study of microemulsion (Smix 1:1; oil: Smix 1:9)-liposome mixture (Lipid: PF127; 100:4).

**Figure 6.** Permeation study on porcine cornea by coumarin-6 loaded liposomes (DIC filter image left; FITC filter image right).

**Figure 7.** Cellular internalisation study of coumarin-6 loaded liposomes on HCEC cell lines (DIC filter image left; FITC filter image right).

**Figure 8.** Dexamethasone release profile.

**Figure 9:** Cytotoxicity study of Liposome (L).

**Figure 10:** Cytotoxicity study of Microemulsion (M).

**Figure 11:** (a) Untreated control HCEC cells (DIC mode), (b) control (FITC mode), (c) Coumarin 6-labelled liposome treated cells (DIC mode) (d) Coumarin 6- labelled liposome treated cells (FITC mode).

**Figure 12:** (a) Untreated control cornea (DIC mode), (b) control (FITC mode), (c) Fluorescent labelled liposome treated cornea (DIC mode) (e) Fluorescent (coumarin 6)-labelled liposome treated cornea (FITC mode) (e) Fluorescent (DAPI)-labelled liposome treated cornea (FITC mode).

**Figure 13:** Flow chart for process scale-up by High Pressure Homogenisation (1 Litre ; 200 eye drop bottle production).

**Figure 14:** DLS micrograph of the liposomal sample prepared by high pressure homogenization.

**Figure 15:** Comparative DLS micrographs and drug content of sterilised and non-sterilised pilot-scale samples prepared by high pressure homogenisation.

**Figure 16:** Fluorescence micrographs of transverse section of cornea treated with coumarin-6 loaded liposomes.

**Figure 17:** Whole eye permeation study from porcine cornea.

**Figure 18:** Six months stability study of Laboratory scale samples (Hydrodynamic diameter and Zeta potential).

**Figure 19:** Six months stability study of Laboratory samples showing drug **content** (%Drug retained).

**Figure 20:** Six months stability study of samples prepared by high pressure homogenisation (Hydrodynamic diameter and Zeta potential).

**Figure 21:** Six months stability study of samples prepared by high pressure homogenisation showing drug content (% Drug retained).

**Figure 22:** Release study comparison of the samples prepared by laboratory method, and

**Figure 23:** Release study comparison of the samples prepared by high pressure homogenisation.

**Examples**

**Design and Development of Liposomal-Microemulsion blend formulation as Mucofiltration Platform technology**

**Summary**

The description involves the preparation of microemulsion containing corticosteroid loaded liposomes. Microemulsion (o/w) composition includes omega-3-fatty acid i.e. $\alpha$-linolenic acid as oil component in the presence of polysorbate 80 as surfactant and PEG 400 as co-surfactant. The liposomes are vesicles prepared from phospholipid (Phospholipon G®) and cholesterol loaded with corticosteroid dexamethasone.
The formulation was prepared by mixing microemulsion formulation with Liposomes containing dexamethasone. The final formulation contains $\alpha$-linolenic acid (0.2% v/v), dexamethasone (0.1 % w/v), polysorbate 80 (0.9%v/v), PEG 400 (0.9 %v/v), Phospholipon® G (2.4% w/v) and cholesterol (0.294% w/v).
Provided is a fast soothing effect to the patient by immediate release of natural oil and then a prolonged release of corticosteroid for inflammation relief. The $\alpha$-linolenic acid, an omega-3-fatty acid constituent of the formulation has been described to be involved in meibomian gland regeneration and an important therapeutic for keratoconjunctivitis sicca. This oil can also play a role in stabilising the tear film by preventing evaporative loss of the aqueous layer. The formulation prepared consisted of nanoparticles in the size range of ~30 nm as characterized by dynamic light scattering. In vitro release profile for dexamethasone was studied over a period of 30 h. The permeation capability of the nanoparticles was assessed on excised porcine corneas by fluorescence microscopy imaging. The *in-vitro* cell permeation was carried out on Human corneal epithelial cells. Finally permeation of the Dexamethasone loaded formulation was studied across excised porcine corneas and enucleated whole eye ball.
The present description provides an innovative approach in the development of an eye drop based formulation that can overcome the problems (i.e. decline in concentration of drug below therapeutic index, repeated dosage requirement and systemic toxicity), faced in conventional marketed alternatives. An innovative approach has been adopted in the present formulation which can act as a mucofiltrating platform for a range of drugs being recommended for various ocular conditions. The idea behind the present platform formulation technology is to include all the elements required for dry eye therapy in the form of a lipid containing nanoparticulate sustained release formulation containing an anti-inflammatory drug along with a natural omega-3-fatty acid supplement ($\alpha$-linolenic acid) for the regeneration of Meibomian gland thus addressing the issues associated with MGD. Further the discussed nanoparticulate can traverse through the mucin mesh pores and can go towards the corneal membrane and later fuse with it by lipid exchange acting as drug eluting reservoir to enhance permeation of drug across cornea.
Meibomian glands are located behind the eyelids and produce necessary fats for tears preventing them from evaporation. Thus the lipids secreted by meibomian gland are required for ocular surface integrity by stabilising tear film. Chronic inflammation disturbs the production and secretion of the lipids emitted by the Meibomian glands. The quality of this lipid mixture is changed, making it stiffer and more viscous. As a result, the lipids cease to effectively protect the tears and eye surface, resulting in familiar dry eye symptoms.
Particularly omega-3-fatty acids have been suggested to inhibit the levels of inflammatory cytokines and hence inflammation within the Meibomian gland in addition to improving the quality of lipids produced by latter making it become more fluid like in nature. Various clinical trials involving omega-3-fatty acid oral supplementation have demonstrated the applicability and effectiveness of omega-3-fatty acids in Meibomian gland regeneration. Recent studies with topical application of omega-3-fatty acids in desiccating Stress-induced dry eye in mice showed improved corneal irregularity and corneal epithelial barrier disruption, and decrease in inflammatory cytokines and oxidative stress markers on the ocular surface. All these studies have clearly supported the therapeutic capability of omega-3-fatty acids in dry eye. Therefore, the microemulsified $\alpha$-linolenic acid constituent of the present formulation will provide an added advantage for symptom relief and therapy of the dry eye disease. Further, along with liposomal dexamethasone constituent the formulation will be first ever novel topically applied approach for dry eye disease and thus can reduce the dosage required as compared to when used alone and an enhanced therapeutic efficacy will be provided.

The mucus layer is a hard barrier to cross and most of the foreign particulate materials and conventionally developed dug delivery particles are efficiently trapped in human mucus layers by steric obstruction and/or adhesion. Trapped particles are typically removed from the mucosal tissue within seconds to a few hours depending on anatomical location, thereby strongly limiting the duration of sustained drug delivery locally. Healthy human mucus viscosity has been reported to be nearly 1000-10,000 times higher than the viscosity of water (at low shear rates). However a size dependent diffusion of molecules has been observed through mucus, where it has been reported that viruses as large as 30 nm can diffuse through the cervical mucin. Norwalk (38 nm) and human papilloma virus (HPV; 55 nm) penetrated mucus at rates roughly equivalent to that in water. Therefore, if particle size can be reduced sufficiently lower than mucin mesh size, a delivery carrier can successfully travel through the low viscosity mucin pores and get in contact with the underlying corneal membrane [30].

The liposomal-dexamethasone component utilised here has been designed to carry near neutral charge. Even the complete formulation is neutral by coating the liposomal component with PF-127 polymer. Further the size of the liposome formulation is ~30 nm. The nanoparticles are non-interacting with mucin in order to enable them to make their way directly towards the corneal membrane passing through the mucin mesh rather than being mucoadhesive and releasing the drug away from the corneal membrane.

The material developed can play a vital role for the treatment of chronic inflammatory ocular conditions including dry eye syndrome, conjunctivitis, uveitis and post-cataract operation etc. Further the formulation can also be explored as a topically applied dosage for posterior segment of eye diseases with a range of other drugs currently recommended for ocular conditions.

The application of the developed eye-drop based ophthalmic drug formulation would be in situations where prolonged retention of a therapeutic agent is needed or in such circumstances where a therapeutic agent itself is not capable to by-pass ophthalmic barriers due to rapid clearance following high tear turnout. Further, the formulation can be used in acute ocular conditions related to anterior and posterior segment diseases. The composition also allows the developed platform technology to deliver omega-3-fatty acids along with a range of hydrophobic drugs including corticosteroids for disease related to meibomian gland later causing ocular inflammation.

1. Liposome suspended in microemulsion-based formulation developed.

a) Liposome development: The liposomes were developed using the thin film hydration method. The lipid chosen was soya lecithin (Phospholipon 90 G) along with cholesterol in varying ratios i.e. 1:1, 2:1, 3:1 and 4:1 and 5:1. The liposomes were prepared with 3 mg dexamethasone. The thin layer was prepared by dissolving lipid, cholesterol and dexamethasone in chloroform/methanol (3:1) and evaporating at 50°C on a rotary evaporator. The thin film was then hydrated with water (10 ml). The various standardisation parameters are presented in Tables 1 and 2.

Table 1: Liposome batches at parameters showing hydrodynamic diameter, PDI, zeta potential and dexamethasone encapsulation efficiency.

| S. No. | PC:Chol (molar ratio) | Dexamethasone (mg) | Chloroform: Methanol(4:1) | Hydration volume (ml) | Hydrodynamic diameter d50(nm) | PDI | Zeta potential (mv) | EE (%) |
|---|---|---|---|---|---|---|---|---|
| 1 | 1:1 | 3 | 5 | 10 | 28.60±23.72 | 0.30 | -20.4 | 79.77 |
| 2 | 2:1 | 3 | 5 | 10 | 25.85±18.94 | 0.14 | -12.1 | 78.99 |
| 3 | 3:1 | 3 | 5 | 10 | 43.8±54.20 | 0.12 | -14.0 | 98.84 |
| 4 | 4:1 | 3 | 5 | 10 | 47.4±65.20 | 0.32 | -12.8 | 98.76 |
| 5 | 5:1 | 3 | 5 | 10 | 441100 | 0.49 | -14.3 | 98.65 |

Table 2. Liposome batches at parameters showing hydrodynamic diameter, PDI, zeta potential and dexamethasone encapsulation efficiency.

| S. No. | PC: Chol: (molar ratio) | Dexamethasone (mg) | Chloroform: Methanol(4:1) | Hydration volume (ml) | Hydrodynamic diameter d50 (nm) | PDI | Zeta potential (mv) | EE (%) |
|---|---|---|---|---|---|---|---|---|
| 1 | 4:1 | 5.08 | 5 | 10 | 202.1±413 | 1.03 | -20.2 | 74.09 |
| 3 | 4:1 | 6.98 | 5 | 10 | 48.4±75.90 | 0.19 | -14.4 | 58.24 |
| 4 | 4:1 | 10.89 | 5 | 10 | 83.9±227.6 | 0.83 | -13 | 34.92 |
| 4 | 4:1 | 15.44 | 5 | 10 | 61.7±82.30 | 0.49 | -14.9 | 24.89 |
| 5 | 4:1 | 19.94 | 5 | 10 | 42±39.30 | 0.1 | -12 | 18.97 |

**b) Microemulsion Preparation:** Mixtures of surfactant and co-surfactant (Smix) were prepared in the weight ratios from 1:1, 2:1, 3:1, 4:1, 1:2 and used as stocks for mixing with oil in different proportions. The Smix ratios were selected in increasing concentration of surfactant with respect to co-surfactant (1:1, 2:1, 3:1, 4:1) and increasing concentration of co-surfactant with respect to surfactant (1:1 and 1:2) to evaluate the phase behaviour. Smix was mixed with oil in different weight ratios (Oil:Smix) i.e. 1:9, 1:8, 1:7, 1:6, 1:5, 1:4, and 1:3. The composition of the various microemulsions prepared are given in table 3 to 9.

Table 3. Change in composition with incremental addition of water with oil to Smix ratio of 1:3.

| S. No. | Oil (μl) | Smix (μl) | Water (μl) | Water added (μl) | Total (μl) | Oil (%) | Smix (%) | Water (%) |
|---|---|---|---|---|---|---|---|---|
| 1 | 4 | 12 | 1 | 1 | 17 | 23.53 | 70.56 | 5.88 |
| 2 | 4 | 12 | 3 | 2 | 19 | 21.05 | 63.156 | 15.78 |
| 3 | 4 | 12 | 4 | 1 | 20 | 20 | 60 | 20 |
| 4 | 4 | 12 | 6 | 2 | 22 | 18.18 | 54.546 | 27.27 |
| 5 | 4 | 12 | 9 | 3 | 25 | 16 | 48 | 36 |
| 6 | 4 | 12 | 12 | 3 | 28 | 14.28 | 42.85 | 42.85 |
| 7 | 4 | 12 | 15 | 3 | 31 | 12.90 | 38.70 | 48.38 |
| 8 | 4 | 12 | 20 | 5 | 36 | 11.11 | 33.33 | 55.55 |
| 9 | 4 | 12 | 27 | 7 | 43 | 9.30 | 27.90 | 62.79 |
| 10 | 4 | 12 | 37 | 10 | 53 | 7.54 | 22.64 | 69.81 |
| 11 | 4 | 12 | 54 | 17 | 70 | 5.71 | 17.14 | 77.14 |
| 12 | 4 | 12 | 84 | 30 | 100 | 4 | 12 | 84 |
| 13 | 4 | 12 | 161 | 77 | 177 | 2.26 | 6.77 | 90.96 |
| 14 | 4 | 12 | 784 | 623 | 800 | 0.5 | 1.5 | 98 |

Table 4. Change in composition with incremental addition of water with oil to Smix ratio of 1:4.

| S. No. | Oil (μl) | Smix (μl) | Water (μl) | Water added (μl) | Total (μl) | Oil (%) | Smix (%) | Water (%) |
|---|---|---|---|---|---|---|---|---|
| 1 | 4 | 16 | 2 | 2 | 22 | 18.18 | 72.72 | 9.09 |
| 2 | 4 | 16 | 3 | 1 | 23 | 17.39 | 69.56 | 13.04 |
| 3 | 4 | 16 | 5 | 2 | 25 | 16 | 64 | 20 |
| 4 | 4 | 16 | 8 | 3 | 28 | 14.28 | 57.14 | 28.57 |
| 5 | 4 | 16 | 11 | 3 | 31 | 12.90 | 51.61 | 35.48 |
| 6 | 4 | 16 | 14 | 3 | 34 | 11.76 | 47.05 | 41.17 |
| 7 | 4 | 16 | 19 | 5 | 39 | 10.25 | 41.02 | 48.71 |
| 8 | 4 | 16 | 25 | 6 | 45 | 8.88 | 35.55 | 55.55 |

(continued)

| S. No. | Oil (μl) | Smix (μl) | Water (μl) | Water added (μl) | Total (μl) | Oil (%) | Smix (%) | Water (%) |
|---|---|---|---|---|---|---|---|---|
| 9 | 4 | 16 | 34 | 9 | 54 | 7.40 | 29.62 | 62.96 |
| 10 | 4 | 16 | 47 | 13 | 67 | 5.97 | 23.88 | 70.14 |
| 11 | 4 | 16 | 67 | 20 | 87 | 4.59 | 18.39 | 77.01 |
| 12 | 4 | 16 | 105 | 38 | 125 | 3.2 | 12.8 | 84 |
| 13 | 4 | 16 | 202 | 97 | 222 | 1.80 | 7.20 | 90.99 |
| 14 | 4 | 16 | 980 | 778 | 1000 | 0.4 | 1.6 | 98 |

Table 5. Change in composition with incremental addition of water with oil to Smix ratio of 1:5.

| S. No. | Oil (μl) | Smix (μl) | Water (μl) | Water added (μl) | Total (μl) | Oil (%) | Smix (%) | Water (%) |
|---|---|---|---|---|---|---|---|---|
| 1 | 2 | 10 | 1 | 1 | 13 | 15.38 | 76.92 | 7 |
| 2 | 2 | 10 | 2 | 1 | 14 | 14.28 | 71.42 | 14 |
| 3 | 2 | 10 | 3 | 1 | 15 | 13.33 | 66.66 | 21 |
| 4 | 2 | 10 | 5 | 2 | 17 | 11.76 | 58.82 | 28 |
| 5 | 2 | 10 | 6 | 1 | 18 | 11.11 | 55.55 | 35 |
| 6 | 2 | 10 | 9 | 3 | 21 | 9.52 | 47.61 | 42 |
| 7 | 2 | 10 | 12 | 3 | 24 | 8.33 | 41.66 | 49 |
| 8 | 2 | 10 | 15 | 3 | 27 | 7.40 | 37.03 | 56 |
| 9 | 2 | 10 | 20 | 5 | 32 | 6.25 | 31.25 | 63 |
| 10 | 2 | 10 | 28 | 8 | 40 | 5 | 25 | 70 |
| 11 | 2 | 10 | 40 | 12 | 52 | 3.84 | 19.23 | 77 |
| 12 | 2 | 10 | 63 | 23 | 75 | 2.66 | 13.33 | 84 |
| 13 | 2 | 10 | 121 | 58 | 133 | 1.50 | 7.51 | 91 |
| 14 | 2 | 10 | 600 | 479 | 612 | 0.32 | 1.63 | 98 |

Table 6. Change in composition with incremental addition of water with oil to Smix ratio of 1:6.

| S. No. | Oil (μl) | Smix (μl) | Water (μl) | Water added (μl) | Total (μl) | Oil (%) | Smix (%) | Water (%) |
|---|---|---|---|---|---|---|---|---|
| 1 | 4 | 24 | 2 | 2 | 30 | 13.33 | 80 | 6.66 |
| 2 | 4 | 24 | 5 | 3 | 33 | 12.12 | 72.72 | 15.15 |
| 3 | 4 | 24 | 7 | 2 | 35 | 11.42 | 68.57 | 20 |
| 4 | 4 | 24 | 11 | 4 | 39 | 10.25 | 61.53 | 28.20 |
| 5 | 4 | 24 | 15 | 4 | 43 | 9.30 | 55.81 | 34.88 |
| 6 | 4 | 24 | 20 | 5 | 48 | 8.33 | 50 | 41.66 |
| 7 | 4 | 24 | 27 | 7 | 52 | 7.27 | 43.63 | 49.09 |
| 8 | 4 | 24 | 36 | 9 | 64 | 6.25 | 37.5 | 56.25 |
| 9 | 4 | 24 | 48 | 12 | 76 | 5.26 | 31.57 | 63.15 |
| 10 | 4 | 24 | 65 | 17 | 93 | 4.30 | 25.80 | 69.89 |
| 11 | 4 | 24 | 94 | 29 | 112 | 3.27 | 19.67 | 77.04 |
| 12 | 4 | 24 | 147 | 53 | 175 | 2.28 | 13.71 | 84 |
| 13 | 4 | 24 | 283 | 136 | 311 | 1.28 | 7.71 | 90.99 |

(continued)

| S. No. | Oil (μl) | Smix (μl) | Water (μl) | Water added (μl) | Total (μl) | Oil (%) | Smix (%) | Water (%) |
|---|---|---|---|---|---|---|---|---|
| 14 | 4 | 24 | 1372 | 1089 | 1400 | 0.28 | 1.71 | 98 |

Table 7. Change in composition with incremental addition of water with oil to Smix ratio of 1:7.

| S. No. | Oil (μl) | Smix (μl) | Water (μl) | Water added (μl) | Total (μl) | Oil (%) | Smix (%) | Water (%) |
|---|---|---|---|---|---|---|---|---|
| 1 | 4 | 28 | 2 | 2 | 34 | 11.76 | 82.35 | 5.88 |
| 2 | 4 | 28 | 5 | 3 | 37 | 10.81 | 75.67 | 13.51 |
| 3 | 4 | 28 | 8 | 3 | 40 | 10 | 70 | 20 |
| 4 | 4 | 28 | 12 | 4 | 44 | 9.09 | 63.63 | 27.27 |
| 5 | 4 | 28 | 17 | 5 | 49 | 8.16 | 57.14 | 34.69 |
| 6 | 4 | 28 | 23 | 6 | 55 | 7.27 | 50.90 | 41.81 |
| 7 | 4 | 28 | 31 | 8 | 63 | 6.34 | 44.44 | 49.20 |
| 8 | 4 | 28 | 41 | 10 | 73 | 5.47 | 38.35 | 56.16 |
| 9 | 4 | 28 | 54 | 13 | 86 | 4.65 | 32.55 | 62.79 |
| 10 | 4 | 28 | 75 | 21 | 107 | 3.73 | 26.16 | 70.09 |
| 11 | 4 | 28 | 107 | 32 | 139 | 2.87 | 20.14 | 76.97 |
| 12 | 4 | 28 | 168 | 61 | 200 | 2 | 14 | 84 |
| 13 | 4 | 28 | 324 | 156 | 356 | 1.12 | 7.86 | 91.01 |
| 14 | 4 | 28 | 1568 | 1244 | 1600 | 0.25 | 1.75 | 98 |

Table 8. Change in composition with incremental addition of water with oil to Smix ratio of 1:8.

| S. No. | Oil (μl) | Smix (μl) | Water (μl) | Water added (μl) | Total (μl) | Oil (%) | Smix (%) | Water (%) |
|---|---|---|---|---|---|---|---|---|
| 1 | 4 | 32 | 3 | 3 | 39 | 10.25 | 82.05 | 7.69 |
| 2 | 4 | 32 | 6 | 3 | 42 | 9.52 | 76.19 | 14.28 |
| 3 | 4 | 32 | 10 | 4 | 46 | 8.69 | 69.56 | 21.73 |
| 4 | 4 | 32 | 14 | 4 | 50 | 8 | 64 | 28 |
| 5 | 4 | 32 | 19 | 5 | 55 | 7.27 | 58.18 | 34.54 |
| 6 | 4 | 32 | 26 | 7 | 62 | 6.45 | 51.61 | 41.93 |
| 7 | 4 | 32 | 35 | 9 | 71 | 5.63 | 45.07 | 49.29 |
| 8 | 4 | 32 | 46 | 11 | 82 | 4.87 | 39.02 | 56.09 |
| 9 | 4 | 32 | 61 | 15 | 97 | 4.12 | 32.98 | 62.88 |
| 10 | 4 | 32 | 84 | 23 | 120 | 3.33 | 26.66 | 70 |
| 11 | 4 | 32 | 121 | 37 | 157 | 2.54 | 20.38 | 77.07 |
| 12 | 4 | 32 | 189 | 68 | 225 | 1.77 | 14.22 | 84 |
| 13 | 4 | 32 | 364 | 175 | 400 | 1 | 8 | 91 |
| 14 | 4 | 32 | 1764 | 1400 | 1800 | 0.22 | 1.77 | 98 |

Table 9. Change in composition with incremental addition of water with oil to Smix ratio of 1:9.

| S. No. | Oil (μl) | Smix (μl) | Water (μl) | Water added (μl) | Total (μl) | Oil (%) | Smix (%) | Water (%) |
|---|---|---|---|---|---|---|---|---|
| 1 | 2 | 18 | 2 | 2 | 22 | 9.09 | 81.81 | 9.09 |
| 2 | 2 | 18 | 3 | 1 | 23 | 8.69 | 78.26 | 13.04 |

(continued)

| S. No. | Oil (μl) | Smix (μl) | Water (μl) | Water added (μl) | Total (μl) | Oil (%) | Smix (%) | Water (%) |
|---|---|---|---|---|---|---|---|---|
| 3 | 2 | 18 | 5 | 2 | 25 | 8 | 72 | 20 |
| 4 | 2 | 18 | 8 | 3 | 28 | 7.14 | 64.28 | 28.57 |
| 5 | 2 | 18 | 11 | 3 | 31 | 6.45 | 58.06 | 35.48 |
| 6 | 2 | 18 | 18 | 7 | 38 | 5.26 | 47.36 | 47.36 |
| 7 | 2 | 18 | 19 | 1 | 39 | 5.12 | 46.15 | 48.71 |
| 8 | 2 | 18 | 25 | 6 | 45 | 4.44 | 40 | 55.55 |
| 9 | 2 | 18 | 34 | 9 | 54 | 3.70 | 33.33 | 62.96 |
| 10 | 2 | 18 | 47 | 13 | 67 | 2.98 | 26.86 | 70.14 |
| 11 | 2 | 18 | 67 | 20 | 87 | 2.29 | 20.68 | 77.01 |
| 12 | 2 | 18 | 105 | 38 | 125 | 1.6 | 14.4 | 84 |
| 13 | 2 | 18 | 202 | 97 | 222 | 0.90 | 8.10 | 90.99 |
| 14 | 2 | 18 | 980 | 778 | 1000 | 0.2 | 1.8 | 98 |

**c) Microemulsion liposome mixture developed with mucopenetrating (PF127 coated) dexamethasone loaded liposomes:** PF127 coated liposomes loaded with dexamethasone were prepared with 1:1:5 molar ratio of Phospholipon G: Cholesterol: PF 127 (20mg:9.8 mg:16 mg) and 4mg dexamethasone. Ethanol injection was used to prepare the liposomes. 500 μl was added to the microemulsion to make up the volume. 1:9 Oil:Smix ratio microemulsion at 4:1, 3:1 and 1:1 Smix (Surf:Co-Surf) composition were used to prepare the mixture.

Table 10. PF127 coated dexamethasone loaded liposomes and various parameters.

| S.no. | PC (mg) | Cholesterol (mg) | PF127 (mg) | PC:PF127 (molar ratio) | Dexamethasone (mg) | Water (ml) | Size (nm) | Zeta potential (mv) | Drug loading per ml (μg) | EE. % |
|---|---|---|---|---|---|---|---|---|---|---|
| 1 | 80 | 9.8 | 0 | 100:0 | 4 | 2 | 19.6 3 | -23.3 | 1422.95 | 71.14 |
| 2 | 80 | 9.8 | 12.8 | 100:1 | 4 | 2 | 32.7 | -11.9 | 1326.73 | 66.33 |
| 3 | 80 | 9.8 | 32 | 100:2.5 | 4 | 2 | 34.7 | -11.7 | 1174.81 | 58.74 |
| 4 | 80 | 9.8 | 51.3 | 100:4 | 4 | 2 | 40.4 | -12.1 | 1593.40 | 79.67 |

**2. Permeation study of the developed formulation on porcine cornea and cellular internalization studies**

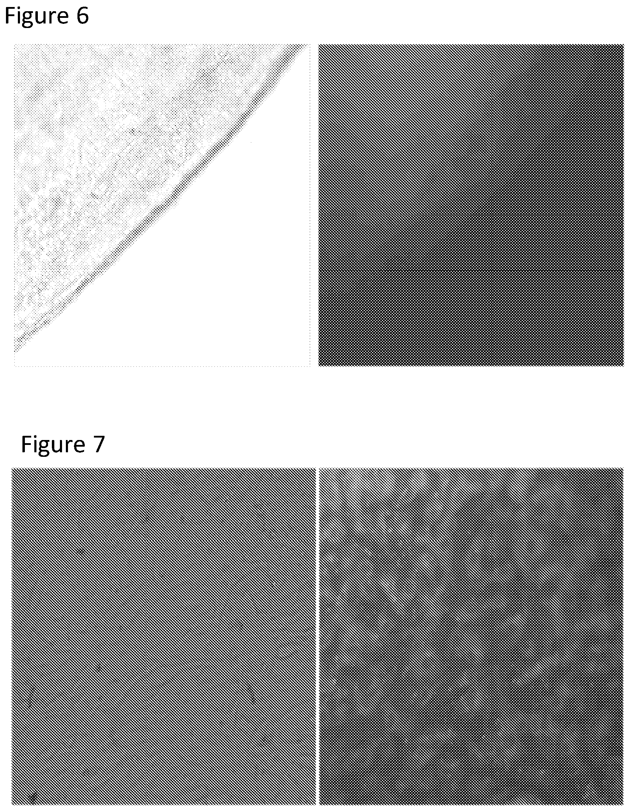

Figures 6 and 7 show the results of a permeation study of the developed formulation on porcine cornea and cellular internalization studies.

**3. Drug Load and release profile established**

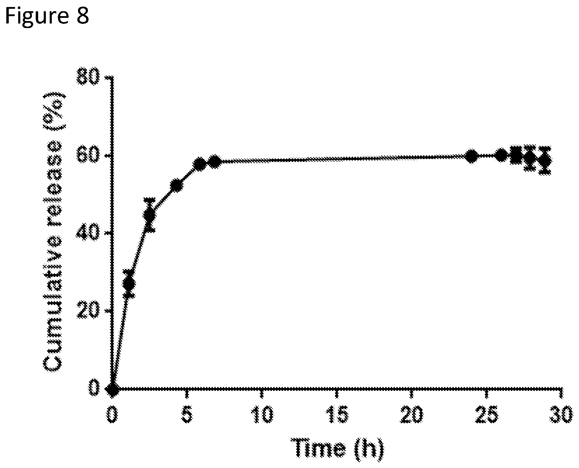

The drug release profile of the microemulsion liposome mixture containing 1mg/ml of dexamethasone was carried out in phosphate buffer saline pH 7.4. The dexamethasone release profile is shown in Figure 8.

The release study was carried out by dialysing 500 μl of 1 mg/ml sample (i.e. 500μg drug) sample against 15 ml release media (PBS pH~7.4 supplemented with 1% v/v tween 80). About 100 μL sample was withdrawn each time and replaced with same volume of fresh media. Samples were analysed on Waters HPLC fitted with C18 column using 50:50 acetonitrile: Phosphoric acid buffer(pH=3). A steady increase in the dexamethasone was observed in first 7h with 60% release, thereafter following a plateau till 30h (Figure 8).

**4. Cytotoxicity study**

The cytotoxicity of the Liposome sample L (Liposome) and M (Microemulsion) was carried out on Human Corneal Epithelium Cell (HCEC). The cells with the initial density of 10,000 cells/well were seeded in a 96-well plate and were then cultured for 24 h in DMEM medium containing 10% FBS. The cells were then treated with varying concentrations of L and M samples. L and M treated cells were incubated in a humidified environment with 5% CO2 at 37 °C for 4 h. After 4 h culture medium is replaced with the fresh medium, the cells were further maintained for another 20 h. After the specified time, MTT reagent was added to each well, and the cells were incubated for another 4 h. The medium in each well was replaced with 200 µL of DMSO to dissolve the formazan crystals. The absorbance (O.D.) was recorded with a microplate reader. The cell viability was calculated by using the following equation:

$$\text{Cell viability (\%) = [O.D. (test)/O.D. (control)] x 100}$$

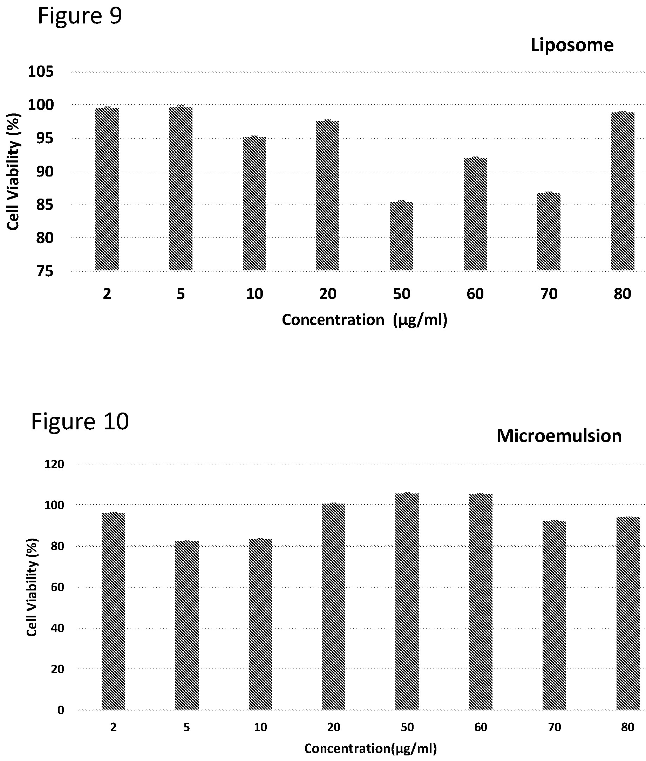

Where O.D. (test) and O.D. (control) are the absorbance values of the cells cultured with and without L/M, respectively. The cytotoxicity study showed that prepared sample L and M is non-toxic up to the given concentrations (Figure 9 and 10).

### 5. Cellular Uptake study

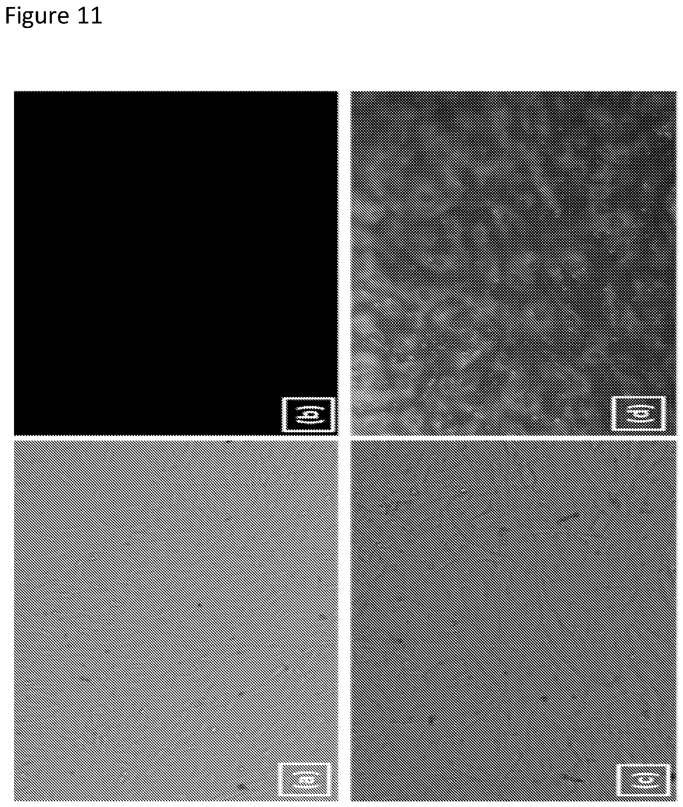

In vitro cellular uptake of Coumarin 6- labelled Liposome sample L (Liposome) were further examined using fluorescent microscopy. The cells were seeded in confocal imaging dishes at a density of $5 \times 104$ cells per dish. The Coumarin 6- labelled Liposome sample L were exposed to the HCEC cells in serum free DMEM medium. After 4 h of incubation at 37 °C, Coumarin 6- labelled Liposome sample L treated serum free medium is removed, and the cells are washed with medium. Further cellular uptake study is performed under fluorescent microscope. The data showed that the Coumarin 6- labelled sample L is successfully internalised in HCEC cells. (Figure 11).

### 6. Mucoadhesion study

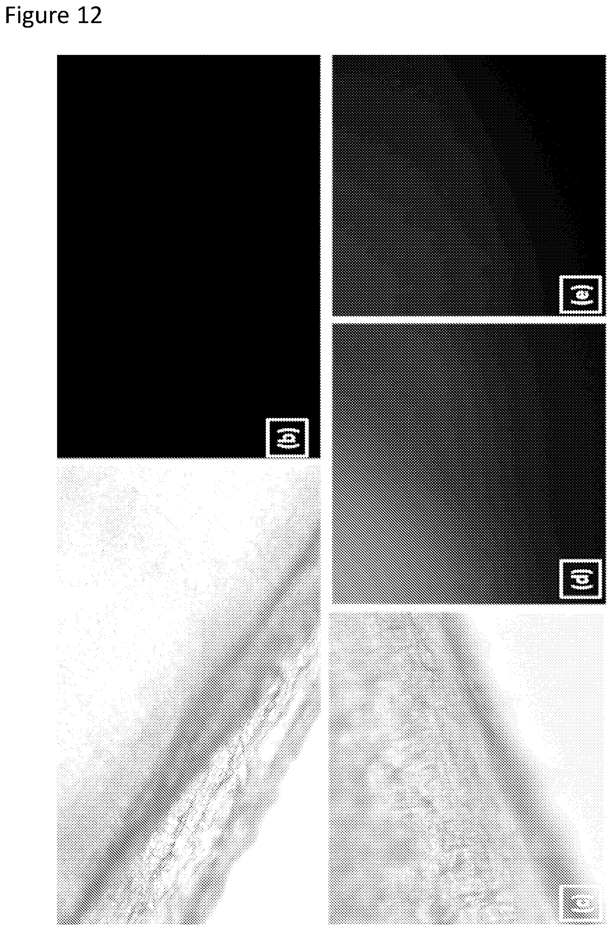

Mucoadhesion study was performed ex vivo with pig cornea. The mucoadhesion study of Coumarin 6- labelled sample L on pig cornea was further examined using fluorescent microscopy. The pig cornea is excised and placed in the confocal imaging dishes containing DMEM medium. The Coumarin 6- labelled Liposome sample L were prepared, and cornea is treated with it in DMEM medium. After 4 h of incubation at 37 °C, Liposome treated serum free medium is removed and the cornea is washed and mucoadhesion study is performed under fluorescent microscope. The mucoadhesion study showed that the Coumarin 6- labelled Liposome sample L are mucoadhesive. As the Coumarin 6- labelled Liposome sample L stick to the cornea (Figure 12).

### 7. Pilot scale optimisation by high pressure homogenisation and sample sterilisation

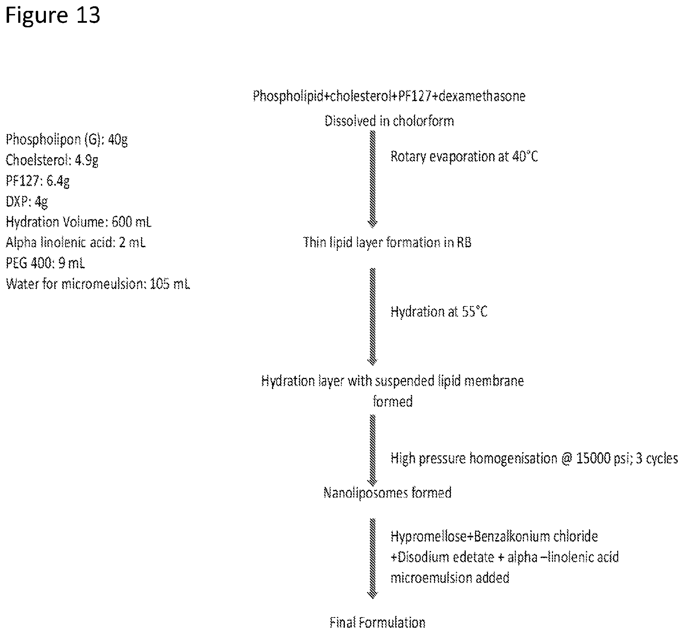

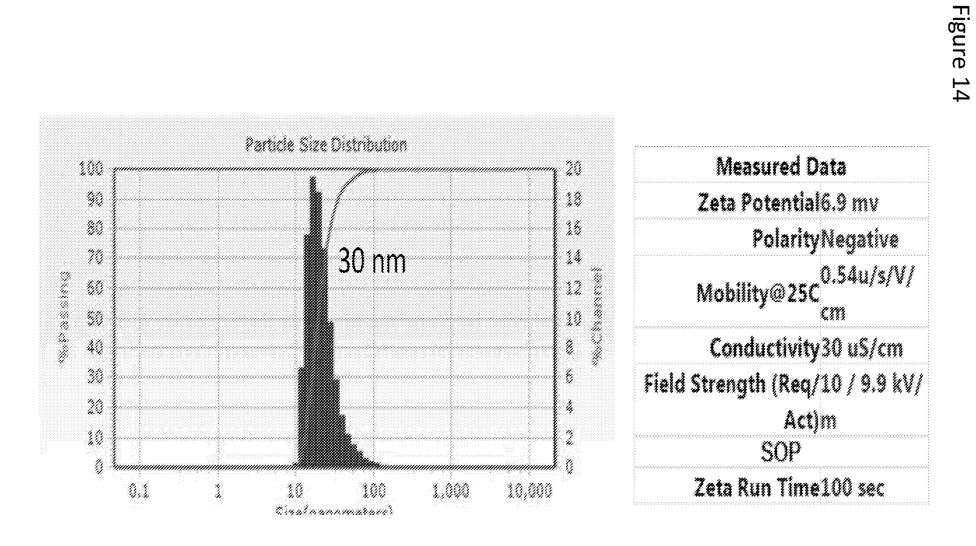

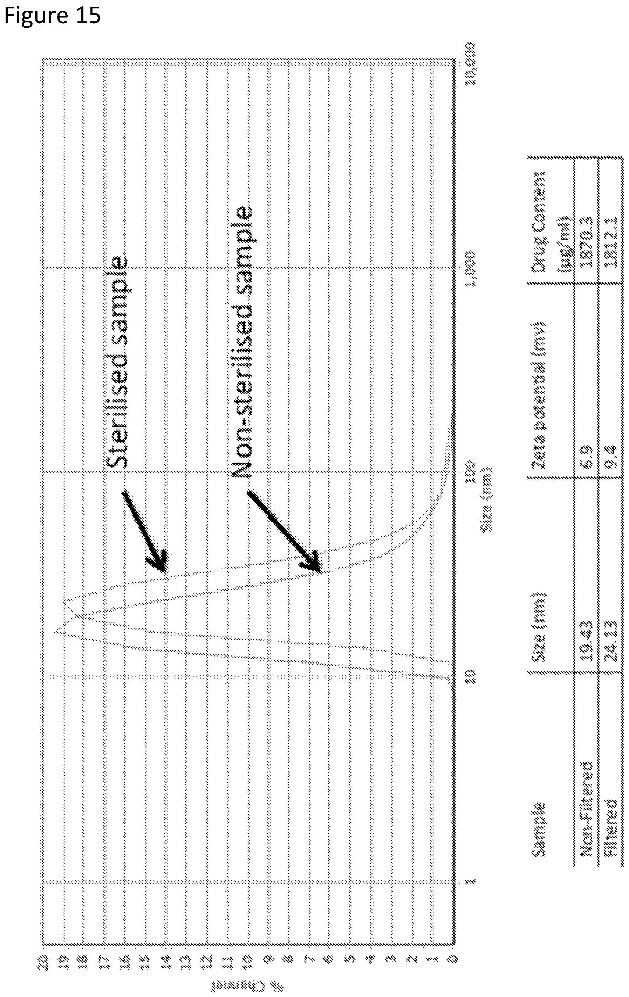

Pilot scale process optimisation was performed according the process flow chart shown in Figure 13. Similar process was carried out as followed in laboratory process except high pressure homogenisation (HPH) was carried out instead of sonication. The formulation was passed through high pressure homogeniser @ 15000 psi; 3 cycles. The hydrodynamic diameter of the pilot scale samples prepared by HPH was observed in the range of 30 nm with 6.9 mv zeta potential. Further no change in size, zeta potential and drug content was observed after sterilisation (Figure 14 and 15).

### 8. Permeation assessment of the formulation on porcine cornea

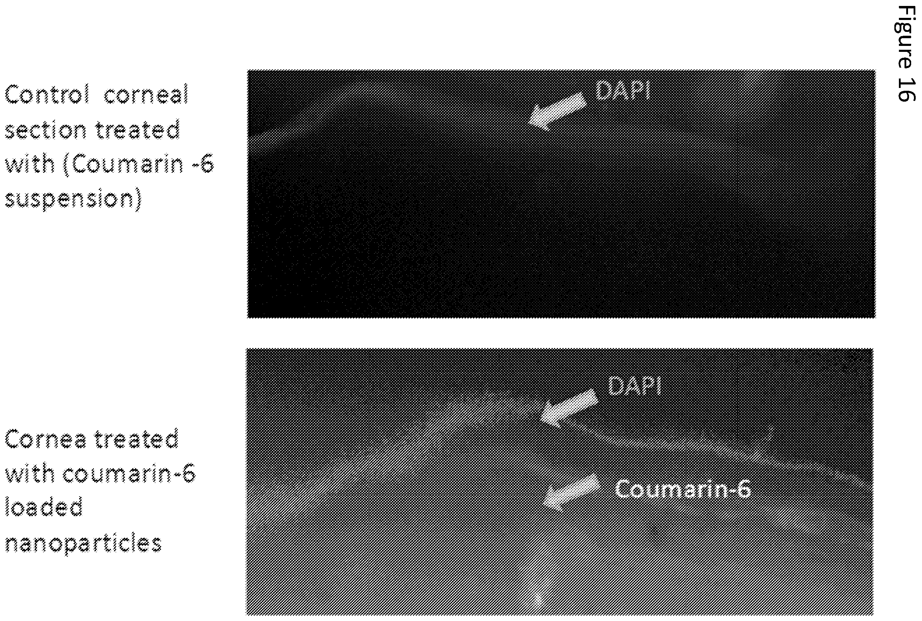

The permeation capability of the liposomes in the deeper layer of cornea was visualised by fluorescence microscopy study of the corneal (Transverse section) samples incubated with coumarin-6 loaded liposomes. Porcine cornea were treated with coumarin-6 loaded liposomes and coumarin-6 suspension as control experiment. After this transverse corneal section were taken and treated with DAPI **(4',6-diamidino-2-phenylindole)** stain. Samples were visualised under DAPI filter and FITC filter and then superimposed in Imaje-J software. The pictures shows transverse corneal sections with upper corneal layer stained with DAPI. In control experiment no green fluorescence was observed in lower corneal layer, however intense coumarin-6 green fluorescence was observed in the one treated with coumarin-6 loaded liposomes. Coumarin-6 loaded liposomes were able to permeate through the deeper corneal layer with surface layer clearly marked by DAPI stain (Figure 16).

### 9. Permeation assessment of the formulation on whole eye

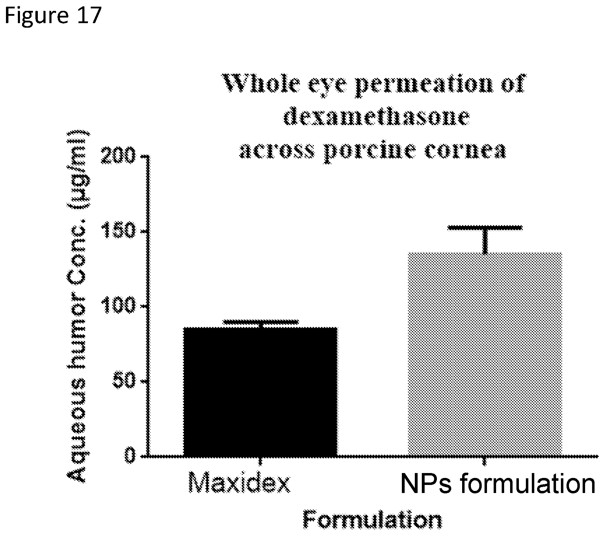

Whole eye permeation study was carried out by fixing the receiver chamber over whole porcine eyes cover the cornea, with the help of a cling film and cellophane tape. About 500 µL of the formulation (0.1 % wt/v; dexamethasone) and Maxidex was added in apical chamber. After 5h eyes were washed with running water and about 100 µL aqueous humor was taken via an injection. Sample was diluted with an equal volume of acetonitrile and analyzed on HPLC. A higher permeation of dexamethasone was observed in aqueous with eyes treated with NPs formulation (135 µg/ml) as compared

to Maxidex (0.1wt/v) (85µg/ml) (Figure 17).

## 10. Stability study of labs scale and pilot scale formulations

Stability studies were performed for both laboratory samples and process scaleup samples over a period of six month. ICH guidelines for refrigerated samples was followed. Studies were carried out at two conditions:

a) 25°C ± 2°C/60% RH ± 5% RH
b) 5°C ± 3°C

Hydrodynamic diameter, Zeta potential and drug content were the criteria selected to assess the stability over six months under predefined conditions.

### a) Laboratory samples

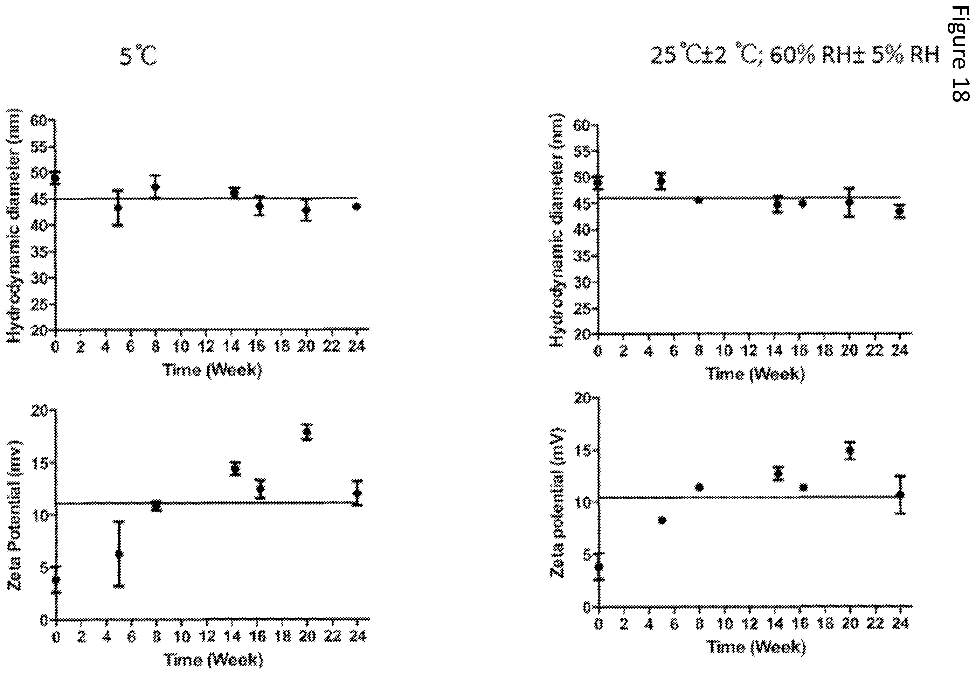

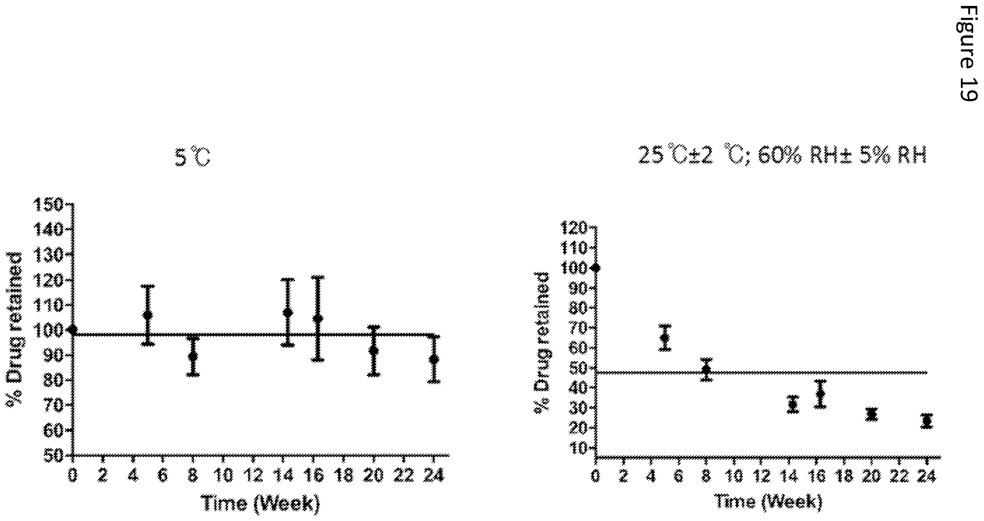

For laboratory samples, no significant variation was observed in hydrodynamic diameter at both storage conditions. Hydrodynamic diameter was observed in the range of 43nm-47 nm at both conditions 5°C. and 25°C. Significant variation was observed in Zeta potential showing a steady increased from 3 mv to 17 mv at both conditions over a period of 24 weeks (Figure 18). Drug retention was found to decrease in the laboratory samples at both storage conditions. Drug retention was decreased by about 10% at 5°C and nearly 76% at 25°C storage condition over a period of 24 weeks (Figure 19).

### b) High pressure homogenisation samples

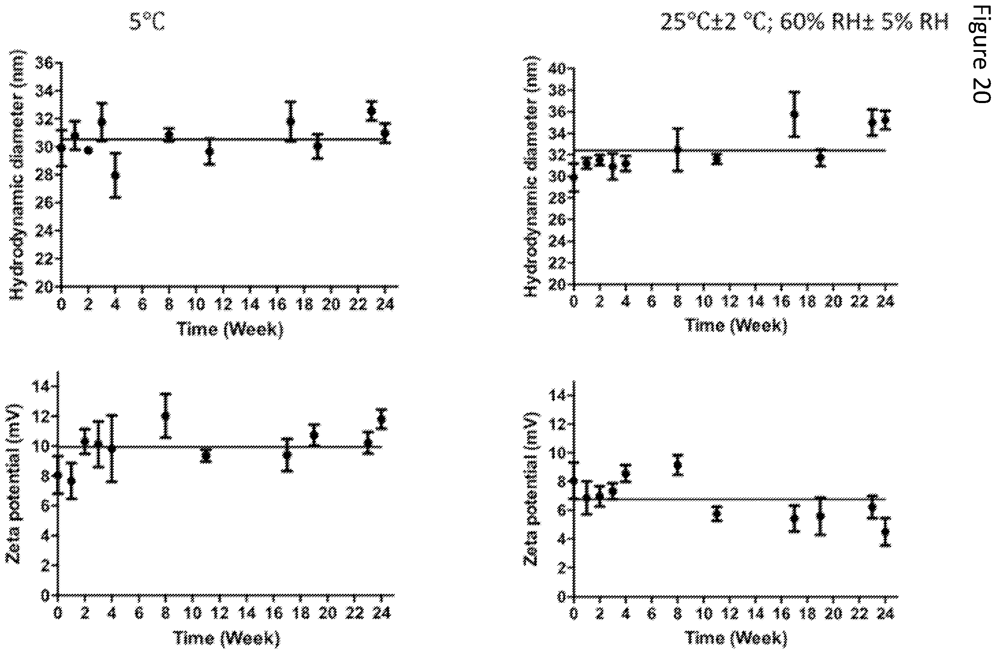

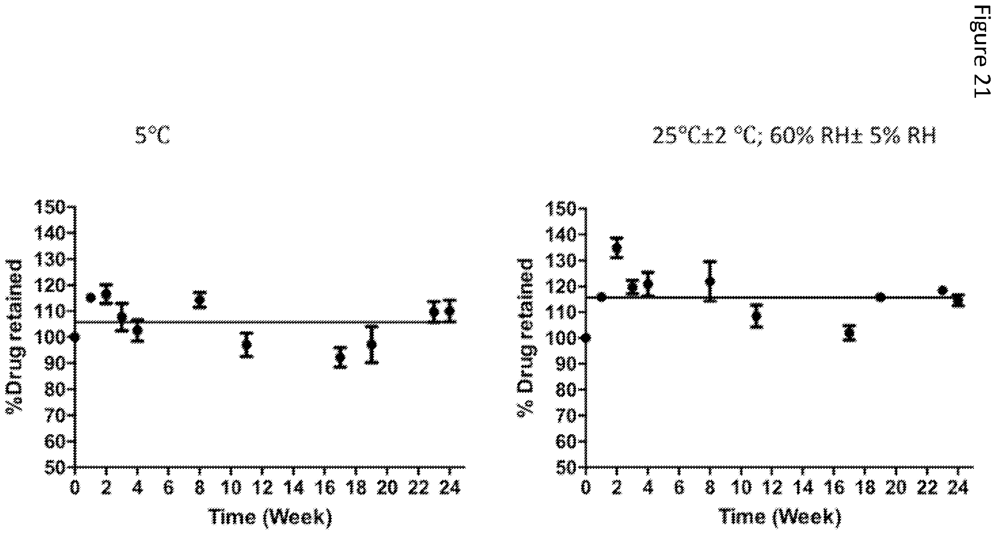

Samples prepared by High pressure homogenisation were found to be quite stable with no significant difference in parameters observed over the period of six months. Size, zeta potential and drug content were quite consistent at both the storage conditions. Hydrodynamic dimeter was observed in the range of 29nm-33nm and zeta potential was in the range of 6 mv-10 mv showing the charge neutrality of the nanoparticles resulting from efficient coating of PF127 polymer. The drug content was also very consistent throughout the study with no observed degradation. No loss of drug was observed at both conditions (Figure 20 and 21).

## 11. Release study comparison of the stability samples prepared by high pressure homogenisation (vs. lab scale comparison)

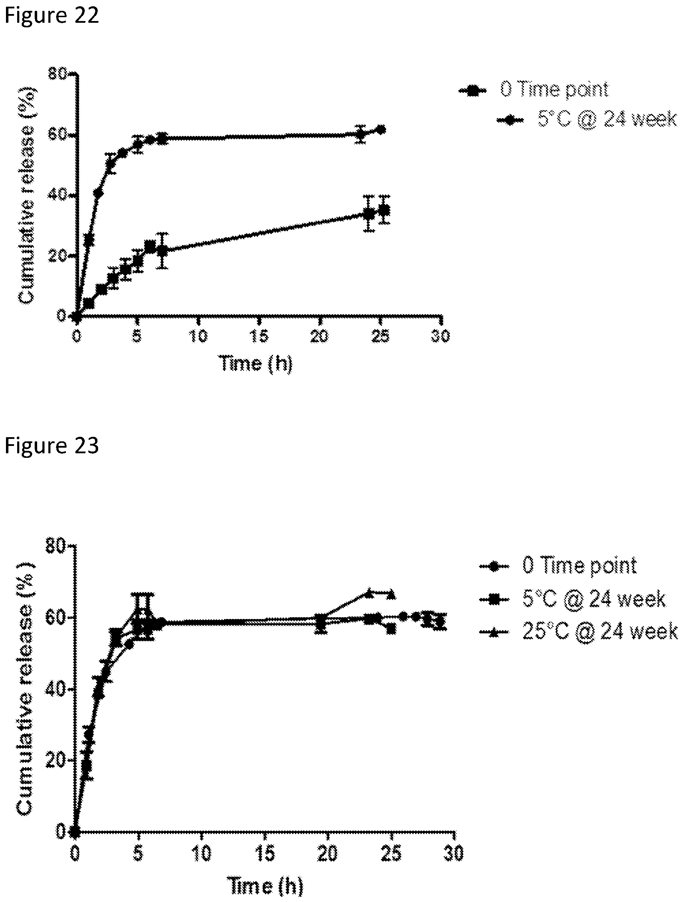

For the lab scale samples, significant variation in release profile of 24 week and initial 0 time point sample was observed. About 60 % drug release was observed in case of 24 week samples whereas it was only 20% in case of 0 time point sample (Figure 22).

For the pilot scale samples, the release study was performed in order to compare to release profile between 0 time point and six-month samples stored at 5°C and 25°C. No significant difference in release was observed between the samples, showing a 60% release in first 7h thereby following a plateau. Therefore, it is quite evident that samples prepared at pilot scale by high pressure homogenisation were relative stable over a period of six month as compared to those prepared at laboratory scale by sonication method (Figure 23).

## References

[11] Lee et al. Lipid-containing lubricants for dry eye: a systematic review. Optometry and Vision Science, 89, 1654-1661, 2012.

[13] Riegeret al. Lipid-containing eye drops: a step closer to natural tears. Ophthalmologica, 201, 206-212, 1990.

[16] Patel et al. World Journal of Pharmacology, 2, 47-64, 2013.

[17] https://www.drugs.com/pro/maxidex.html

[18] http://www.medicines.ie/medicine/15432/SPC/ Lotemax+0.5++Eye+Drops%2C+Suspension/

[19] Kompella et al. Therapeutic Delivery, 1, 435-456, 2010.

[20] http://eyetubeod.com/2017/04/dry-eye-disease-therapy-a-flowing-pipeline/

[21] http://kalarx.com/technology/focus-on-eye-care/

[22] Schopf et al. Ocular pharmacokinetics of a novel Loteprednol Etabonate 0.4% ophthalmic formulation. Ophthalmology and Therapy, 3, 63-72, 2014.

[23] http://oculispharma.com/

[24] Shulman et al. Topical dexamethasone-cyclodextrin nanoparticle eye drops for non-infectious Uveitic macular oedema and vitritis - a pilot study. Acta Ophthalmologica, 93, 411-415, 2015.

[25] Jing et al. Emerging Treatment Options for Meibomian Gland Dysfunction. Clinical Ophthalmology 7, 1797-1803, (2013).

[26] Knop et al. The International Workshop on Meibomian Gland Dysfunction: Report of the Subcommittee on Anatomy, Physiology, and Pathophysiology of the Meibomian Gland. Investigative Ophthalmology & Visual Science 52.4, 1938-1978. (2011).

[27] Bhargava et al. A Randomized Controlled Trial of Omega-3 Fatty Acids in Dry Eye Syndrome. International Journal of Ophthalmology 6.6, 811-816, 2013.

[28] Hampel et al. In Vitro Effects of Docosahexaenoic and Eicosapentaenoic Acid on Human Meibomian Gland Epithelial Cells. Journal of Experimental Eye Research. 140, 139-48, 2015.

[29] Li et al. Effects of eye drops containing a mixture of Omega-3 Essential Fatty Acids and Hyaluronic Acid on the ocular surface in desiccating stress-induced murine dry eye. Current Eye Research, 1-8, 2014. DOI: 10.3109/02713683.2014.884595

[30] Lai et al. Mucus-penetrating nanoparticles for drug and gene delivery to mucosal tissues. 61, 158-171, 2009.

[31] Olmsted et al. Diffusion of macromolecules and virus-like particles in human cervical mucus. Biophysical Journal, 81:1930-1937, 2001.

## Claims

1. A composition for ophthalmic delivery of a therapeutic agent, the composition comprising an oil-in-water (o/w) microemulsion comprising a fatty acid, or fatty acid ester, as the oil phase; an aqueous phase; a surfactant; and a co-surfactant, and
   wherein the composition further comprises a suspension of therapeutic agent-loaded nanoparticles in the aqueous phase of the oil-in-water emulsion.

2. The composition according to claim 1, wherein the oil phase comprises omega-3-fatty acid,
   optionally wherein the omega-3-fatty acid comprises or consists of $\alpha$-linolenic acid

3. The composition according to any preceding claim, wherein the oil phase is in an amount of between 0.1% and 0.5% v/v of the composition.

4. The composition according to any preceding claim, wherein the surfactant is selected from the group comprising lecithin; lecithin derivatives; glycerol fatty acid esters; sorbitan fatty acid esters; polyoxyethylene sorbitan fatty acid esters; propylene glycol; and PEG 200; or combinations thereof, or
   wherein the surfactant comprises or consists of polysorbate 80.

5. The composition according to any preceding claim, wherein the surfactant is in an amount of between 0.1 and 2% v/v of the composition.

6. The composition according to any preceding claim, wherein the co-surfactant comprises or consists of an alkanol; an alkane-diol; an alkane-polyol; PEG 200; PEG 400; DSPE-PEG; poloxamer; and a combination where one component is block copolymer of a glycol monomer and other is polymer conjugated phospholipid; or combinations thereof, optionally wherein the co-surfactant is in an amount of between 0.1 and 2% v/v of the composition.

7. The composition according to any preceding claim, wherein the weight ratio of surfactant to co-surfactant is from 4:1 to 1:2, and/or
   wherein the weight ratio of oil phase and surfactant/co-surfactant mixture is between 1.3 and 1.9.

8. The composition according to any preceding claim, wherein the nanoparticle comprises a nanoparticle selected from the group comprising a liposome; a nanoemulsion; a lipid nanocarrier; a solid lipid nanoparticle; a nanostructured lipid carrier (NLC); a polymeric capsule; and a polymeric nanosphere; or a combination thereof, and/or

   wherein the therapeutic agent-loaded nanoparticle is a liposome, and/or
   wherein the nanoparticles comprise vesicles prepared from phospholipid and another lipid, such as cholesterol, and it is loaded with the therapeutic agent;

optionally wherein the phospholipid is in an amount of between 1% and 5% v/v of the composition.

9. The composition according to claim 8, wherein the phospholipid comprises or consists of lecithin, optionally wherein the phospholipid is in an amount of between 1% and 5% v/v of the composition.

10. The composition according to any preceding claim, wherein the nanoparticle comprises a lipid selected from the group comprising tristearin, stearic acid, cetyl palmitate, cholesterol, glyceryl distearate NF / glyceryl palmitostearate, esters of behenic acid with glycerol, tripalmitin, tristearin, hydrogenated palm oil, cetylpalmitate, glyceryl stearate, glycerol monostearate, glycerol monostearate and PEG-75 stearate, cetyl alcohol and ceteth-20 / steareth-20 and cholesterol; or combinations thereof, and/or

wherein the nanoparticle is a liposome and the liposome bilayer comprises cholesterol, and/or
wherein the nanoparticles are liposomes and are further coated with a polymer or copolymer,
optionally wherein the polymer or copolymer comprise an amphiphilic block copolymer, such as a poloxamer.

11. The composition according to any preceding claim, wherein the therapeutic agent is suitable for treatment or prevention of an eye disorder, and/or

wherein the therapeutic agent is hydrophobic, and/or
wherein the therapeutic agent is an anti-inflammatory agent, antihistamine, decongestant or combinations thereof,
optionally wherein the anti-inflammatory agent comprises or consists of a corticosteroid.

12. The composition according to any preceding claim, wherein the therapeutic agent is in the amount of between 0.01 % and 0.5 % w/v of the composition, and/or
wherein the therapeutic agent is at a concentration of between 0.01 mg/ml and 2 mg/ml.

13. The composition according to any preceding claim, wherein the composition is a pharmaceutically or ophthalmically acceptable composition, and/or
wherein the composition is suitable for topical administration to the eye.

14. A composition according to any preceding claim, for use in the treatment or prevention of an eye disorder in a subject, optionally wherein the use comprises topical administration of the composition to the surface of the eye or to the eyelid.

15. An eye drop dispenser or eye wash device comprising the composition according to any one of claims 1-13.


**Patentansprüche**

1. Zusammensetzung zur ophthalmischen Abgabe eines therapeutischen Wirkstoffs, wobei die Zusammensetzung eine Öl-in-Wasser(Ö-W)-Mikroemulsion, die eine Fettsäure oder ein Fettsäureester umfasst, als die Ölphase; eine wässrige Phase; ein Tensid und ein Cotensid umfasst, und
wobei die Zusammenfassung ferner eine Suspension von therapeutisch wirkstoffbeladenen Nanopartikeln in der wässrigen Phase der Öl-in-Wasser-Emulsion umfasst.

2. Zusammensetzung nach Anspruch 1, wobei die Ölphase eine Omega-3-Fettsäure umfasst,
wobei die Omega-3-Fettsäure optional eine $\alpha$-Linolensäure umfasst oder aus dieser besteht

3. Zusammensetzung nach einem der vorhergehenden Ansprüche, wobei die Ölphase in einer Menge von zwischen 0,1 Vol.-% und 0,5 Vol.-% der Zusammensetzung vorliegt.

4. Zusammensetzung nach einem der vorhergehenden Ansprüche, wobei das Tensid aus der Gruppe umfassend Lecithin; Lecithinderivate; Glycerin-Fettsäureester; Sorbitanfettsäureester; Polyoxyethylen-Sorbitanfettsäureester; Propylenglykol; und PEG 200; oder Kombinationen davon ausgewählt ist, oder
wobei das Tensid Polysorbat 80 umfasst oder aus diesem besteht.

5. Zusammensetzung nach einem der vorhergehenden Ansprüche, wobei das Tensid in einer Menge von zwischen 0,1 und 2 Vol.-% der Zusammensetzung vorliegt.

**6.** Zusammensetzung nach einem der vorhergehenden Ansprüche, wobei das Cotensid ein Alkanol; ein Alkandiol; ein Alkanpolyol; PEG 200; PEG 400; DSPE-PEG; Poloxamer; und eine Kombination, wobei eine Komponente ein Block-Copolymer eines Glycolmonomers ist und eine andere ein polymerkonjugiertes Phospholipid ist; oder Kombinationen davon umfasst oder aus diesen besteht,
wobei das Cotensid optional in einer Menge von zwischen 0,1 und 2 Vol.-% der Zusammensetzung vorliegt.

**7.** Zusammensetzung nach einem der vorhergehenden Ansprüche, wobei das Gewichtsverhältnis von Tensid zu Cotensid von 4:1 und 1:2 vorliegt, und/oder
wobei das Gewichtsverhältnis von Ölphase und Tensid- /Cotensid-Gemisch zwischen 1,3 und 1,9 liegt.

**8.** Zusammensetzung nach einem der vorhergehenden Ansprüche, wobei der Nanopartikel einen Nanopartikel umfasst, der aus der Gruppe umfassend ein Liposom; eine Nanoemulsion; einen Lipid-Nanoträger; einen festen Lipid-Nanopartikel; einen nanostrukturierten Lipidträger (NLC); eine Polymerkapsel; und ein Polymer-Nanokügelchen; oder eine Kombination von diesen ausgewählt ist, und/oder

wobei der therapeutisch wirkstoffbeladene Nanopartikel ein Liposom ist, und/oder
wobei die Nanopartikel Vesikel umfassen, die aus Phospholipid und einem anderen Lipid, wie etwa Cholesterin, hergestellt sind, und mit dem therapeutischen Wirkstoff beladen sind;
wobei das Phospholipid optional in einer Menge von zwischen 1 Vol.-% und 5 Vol.-% der Zusammensetzung vorliegt.

**9.** Zusammensetzung nach Anspruch 8, wobei das Phospholipid Lecithin umfasst oder aus diesem besteht,
wobei das Phospholipid optional in einer Menge von zwischen 1 Vol.-% und 5 Vol.-% der Zusammensetzung vorliegt.

**10.** Zusammensetzung nach einem der vorhergehenden Ansprüche, wobei der Nanopartikel ein Lipid umfasst, das aus der Gruppe umfassend Tristearin, Stearinsäure, Cetylpalmitat, Cholesterin, Glyceryldistearat NF / Glycerylpalmitostearat, Ester der Behensäure mit Glycerin, Tripalmitin, Tristearin, gehärtetem Palmöl, Cetylpalmitat, Glycerylstearat, Glycerinmonostearat, Glycerinmonostearat und PEG-75-Stereat, Cetylalkohol und Ceteth-20 / Steareth-20 und Cholesterin; oder Kombinationen davon ausgewählt ist, und/oder

wobei der Nanopartikel ein Liposom ist und die Liposom-Doppelschicht Cholesterin umfasst, und/oder
wobei die Nanopartikel Liposome sind und ferner mit einem Polymer oder Copolymer beschichtet sind,
wobei das Polymer oder Copolymer optional ein amphiphiles Block-Copolymer, wie etwa Poloxamer, umfasst.

**11.** Zusammensetzung nach einem der vorhergehenden Ansprüche, wobei der therapeutische Wirkstoff zur Behandlung oder Prävention einer Augenerkrankung geeignet ist, und/oder

wobei der therapeutische Wirkstoff hydrophob ist, und/oder
wobei der therapeutische Wirkstoff ein entzündungshemmender Wirkstoff, Antihistamin, abschwellendes Mittel oder Kombinationen davon ist,
wobei der entzündungshemmende Wirkstoff optional ein Kortikosteroid umfasst oder aus diesem besteht.

**12.** Zusammensetzung nach einem der vorhergehenden Ansprüche, wobei der therapeutische Wirkstoff in der Menge von zwischen 0,01 Gew.-% und 0,5 Gew.-% der Zusammensetzung vorliegt, und/oder
wobei der therapeutische Wirkstoff in einer Konzentration von zwischen 0,01 mg/ml und 2 mg/ml vorliegt.

**13.** Zusammensetzung nach einem der vorhergehenden Ansprüche, wobei die Zusammensetzung eine pharmazeutisch oder ophthalmisch unbedenkliche Zusammensetzung ist, und/oder
wobei die Zusammensetzung zur topischen Verabreichung an das Auge geeignet ist.

**14.** Zusammensetzung nach einem der vorhergehenden Ansprüche, zur Verwendung bei der Behandlung oder Prävention einer Augenerkrankung bei einem Subjekt,
wobei die Verwendung optional eine topische Verabreichung der Zusammensetzung an die Oberfläche des Auges oder an das Augenlid umfasst.

**15.** Augentropfenspender oder Augenwaschvorrichtung, umfassend die Zusammensetzung nach einem der Ansprüche 1-13.

**Revendications**

1. Composition pour l'administration ophtalmique d'un agent thérapeutique, la composition comprenant une microémulsion d'huile dans l'eau (h/e) comprenant un acide gras, ou un ester d'acide gras, comme phase huileuse ; une phase aqueuse ; un tensioactif ; et un co-tensioactif, et
dans laquelle la composition comprend en outre une suspension de nanoparticules chargées d'agent thérapeutique dans la phase aqueuse de l'émulsion d'huile dans l'eau.

2. Composition selon la revendication 1, dans laquelle la phase huileuse comprend un acide gras oméga-3, éventuellement dans laquelle l'acide gras oméga-3 comprend ou est constitué d'acide linoléique α.

3. Composition selon l'une quelconque des revendications précédentes, dans laquelle la phase huileuse est présente en une quantité comprise entre 0,1 % et 0,5 % v/v de la composition.

4. Composition selon l'une quelconque des revendications précédentes, dans laquelle le tensioactif est choisi dans le groupe comprenant la lécithine ; les dérivés de la lécithine ; les esters d'acides gras de glycérol ; les esters d'acides gras de sorbitan ; les esters d'acide gras de polyoxyéthylène sorbitane ; le propylène glycol ; et PEG 200 ; ou des combinaisons de ceux-ci, ou
dans laquelle le tensioactif comprend ou est constitué de polysorbate 80.

5. Composition selon l'une quelconque des revendications précédentes, dans laquelle le tensioactif est présent en une quantité comprise entre 0,1 et 2 % v/v de la composition.

6. Composition selon l'une quelconque des revendications précédentes, dans laquelle le co-tensioactif comprend ou est constitué d'un alcanol ; d'un alcane-diol ; d'un alcane-polyol ; de PEG 200 ; de PEG 400 ; de DSPE-PEG ; de poloxamère ; et d'une combinaison dans laquelle un composant est un copolymère séquencé d'un monomère de glycol et l'autre composent est un phospholipide conjugué à un polymère ; ou des combinaisons de ceux-ci, éventuellement dans laquelle le co-tensioactif est présent en une quantité comprise entre 0,1 et 2 % v/v de la composition.

7. Composition selon l'une quelconque des revendications précédentes, dans laquelle le rapport pondéral du tensioactif par rapport au co-tensioactif est de 4 : 1 à 1 : 2, et/ou
dans laquelle le rapport pondéral de la phase huileuse par rapport au mélange tensioactif/co-tensioactif est compris entre 1,3 et 1,9.

8. Composition selon l'une quelconque des revendications précédentes, dans laquelle la nanoparticule comprend une nanoparticule choisie dans le groupe comprenant un liposome ; une nanoémulsion ; un nanovecteur lipidique ; une nanoparticule lipidique solide ; un vecteur lipidique nanostructuré (NLC) ; une capsule polymère ; et une nanosphère polymère ; ou une combinaison de ceux-ci, et/ou

dans laquelle la nanoparticule chargée d'agent thérapeutique est un liposome, et/ou
dans laquelle les nanoparticules comprennent des vésicules préparées à partir de phospholipide et d'un autre lipide, tel que le cholestérol, et sont chargées avec l'agent thérapeutique ;
éventuellement dans laquelle le phospholipide est présent en une quantité comprise entre 1 % et 5 % v/v de la composition.

9. Composition selon la revendication 8, dans laquelle le phospholipide comprend ou est constitué de lécithine, éventuellement dans laquelle le phospholipide est présent en une quantité comprise entre 1 % et 5 % v/v de la composition.

10. Composition selon l'une quelconque des revendications précédentes, dans laquelle la nanoparticule comprend un lipide choisi dans le groupe comprenant la tristéarine, l'acide stéarique, le cétylepalmitate, le cholestérol, le distéarate de glycéryle NF / le palmitostéarate de glycéryle, les esters d'acide béhénique avec le glycérol, la tripalmitine, la tristéarine, l'huile de palme hydrogénée, la cétylpalmitate, le stéarate de glycéryle, le monostéarate de glycérol, le monostéarate de glycérol et le stéarate de PEG-75, alcool cétylique et ceteth-20/steareth-20 et cholestérol ; ou des combinaisons de ceux-ci, et/ou

dans laquelle la nanoparticule est un liposome et la bicouche de liposome comprend du cholestérol, et/ou

dans laquelle les nanoparticules sont des liposomes et sont en outre recouvertes d'un polymère ou d'un copolymère,
éventuellement dans laquelle le polymère ou le copolymère comprend un copolymère séquencé amphiphile, tel qu'un poloxamère.

11. Composition selon l'une quelconque des revendications précédentes, dans laquelle l'agent thérapeutique est adapté au traitement ou à la prévention d'un trouble oculaire, et/ou

dans laquelle l'agent thérapeutique est hydrophobe, et/ou
dans laquelle l'agent thérapeutique est un agent anti-inflammatoire, un antihistaminique, un décongestionnant ou des combinaisons de ceux-ci,
éventuellement dans laquelle l'agent anti-inflammatoire comprend ou est constitué d'un corticostéroïde.

12. Composition selon l'une quelconque des revendications précédentes, dans laquelle l'agent thérapeutique est présent en une quantité comprise entre 0,01 % et 0,5 % p/v de la composition, et/ou
dans laquelle l'agent thérapeutique est à une concentration comprise entre 0,01 mg/ml et 2 mg/ml.

13. Composition selon l'une quelconque des revendications précédentes, dans laquelle la composition est une composition pharmaceutiquement ou ophtalmiquement acceptable, et/ou
dans laquelle la composition est adaptée à une administration topique dans l'œil.

14. Composition selon l'une quelconque des revendications précédentes, destinée à être utilisée dans le traitement ou la prévention d'un trouble oculaire chez un sujet,
l'utilisation comprenant éventuellement l'administration topique de la composition sur la surface de l'œil ou sur la paupière.

15. Distributeur de gouttes oculaires ou dispositif de lavage oculaire comprenant la composition selon l'une quelconque des revendications 1 à 13.

# Figure 1

Figure 2

Figure 3

Figure 4

Figure 5

Figure 6

Figure 7

Figure 8

## Figure 9

**Liposome**

## Figure 10

**Microemulsion**

Figure 11

Figure 12

# Figure 13

Phospholipon (G): 40g
Choelsterol: 4.9g
PF127: 6.4g
DXP: 4g
Hydration Volume: 600 mL
Alpha linolenic acid: 2 mL
PEG 400: 9 mL
Water for micromeulsion: 105 mL

Phospholipid+cholesterol+PF127+dexamethasone
Dissolved in cholorform

Rotary evaporation at 40°C

Thin lipid layer formation in RB

Hydration at 55°C

Hydration layer with suspended lipid membrane
formed

High pressure homogenisation @ 15000 psi; 3 cycles

Nanoliposomes formed

Hypromellose+Benzalkonium chloride
+Disodium edetate + alpha –linolenic acid
microemulsion added

Final Formulation

Figure 14

| | |
|---|---|
| Measured Data | |
| Zeta Potential | 6.9 mv |
| Polarity | Negative |
| Mobility@25C | 0.54u/s/V/cm |
| Conductivity | 30 uS/cm |
| Field Strength (Req/Act) | 10 / 9.9 kV/m |
| SOP | |
| Zeta Run Time | 100 sec |

# Figure 15

| Sample | Size (nm) | Zeta potential (mv) | Drug Content (ug/ml) |
|---|---|---|---|
| Non-Filtered | 19.43 | 6.9 | 1870.3 |
| Filtered | 24.13 | 9.4 | 1812.1 |

Figure 16

Control corneal section treated with (Coumarin -6 suspension)

Cornea treated with coumarin-6 loaded nanoparticles

Figure 17

Whole eye permeation of dexamethasone across porcine cornea

Figure 18

Figure 19

Figure 20

Figure 21

Figure 22

Figure 23

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 104188937 A
- CN 102793672 A
- US 2017296496 A1

**Non-patent literature cited in the description**

- **LEE et al.** Lipid-containing lubricants for dry eye: a systematic review. *Optometry and Vision Science*, 2012, vol. 89, 1654-1661
- **RIEGERET**. Lipid-containing eye drops: a step closer to natural tears. *Ophthalmologica*, 1990, vol. 201, 206-212
- **PATEL et al.** *World Journal of Pharmacology*, 2013, vol. 2, 47-64
- **KOMPELLA et al.** *Therapeutic Delivery*, 2010, vol. 1, 435-456
- **SCHOPF et al.** Ocular pharmacokinetics of a novel Loteprednol Etabonate 0.4% ophthalmic formulation.. *Ophthalmology and Therapy*, 2014, vol. 3, 63-72
- **SHULMAN et al.** Topical dexamethasone-cyclodextrin nanoparticle eye drops for non-infectious Uveitic macular oedema and vitritis - a pilot study. *Acta Ophthalmologica*, 2015, vol. 93, 411-415
- **JING et al.** Emerging Treatment Options for Meibomian Gland Dysfunction.. *Clinical Ophthalmology*, 2013, vol. 7, 1797-1803
- **KNOP et al.** The International Workshop on Meibomian Gland Dysfunction: Report of the Subcommittee on Anatomy, Physiology, and Pathophysiology of the Meibomian Gland.. *Investigative Ophthalmology & Visual Science*, 2011, vol. 52 (4), 1938-1978
- **BHARGAVA et al.** A Randomized Controlled Trial of Omega-3 Fatty Acids in Dry Eye Syndrome.. *International Journal of Ophthalmology*, 2013, vol. 6 (6), 811-816
- **HAMPEL et al.** In Vitro Effects of Docosahexaenoic and Eicosapentaenoic Acid on Human Meibomian Gland Epithelial Cells. *Journal of Experimental Eye Research.*, 2015, vol. 140, 139-48
- **LAI et al.** *Mucus-penetrating nanoparticles for drug and gene delivery to mucosal tissues*, 2009, vol. 61, 158-171
- **OLMSTED et al.** Diffusion of macromolecules and virus-like particles in human cervical mucus.. *Biophysical Journal*, 2001, vol. 81, 1930-1937